# EUROPEAN PATENT SPECIFICATION

(11) **EP 2 976 096 B1**
(45) Date of publication and mention of the grant of the patent: **27.02.2019**
(21) Application number: 14713072.8
(22) Date of filing: 19.03.2014
(51) Int. Cl.: A61K 9/00, A61K 38/28

(54) **INSULIN DOSING REGIMEN**
INSULINDOSIERSCHEMA
RÉGIME DE DOSAGE POUR INSULINE

(30) Priority: 20.03.2013 EP 13160194; 22.03.2013 US 201361804363 P
(43) Date of publication of application: 27.01.2016
(73) Proprietor: Novo Nordisk A/S, 2880 Bagsværd (DK)
(72) Inventor: JOHANSEN, Thue, DK-2880 Bagsvaerd (DK)
(86) International application number: PCT/EP2014/055533
(87) International publication number: WO 2014/147141

(56) References cited:
- WO-A2-2005/012347
- HOEVELMANN U ET AL: "Insulin degludec 200 U/ml is ultra-long-acting and has a flat and stable glucose-lowering effect", DIABETOLOGIA, vol. 55, no. Suppl. 1, October 2012 (2012-10), pages S374-S375, XP002723769, & 48TH ANNUAL MEETING OF THE EUROPEAN-ASSOCIATION-FOR-THE-STUDY-OF-DIAB ETES; BERLIN, GERMANY; OCTOBER 01 -05, 2012
- WANG FEI ET AL: "Insulin degludec as an ultralong-acting basal insulin once a day: a systematic review.", DIABETES, METABOLIC SYNDROME AND OBESITY : TARGETS AND THERAPY 2012, vol. 5, 2012, pages 191-204, XP002723770, ISSN: 1178-7007
- KORSATKO S ET AL: "Ultra-long-acting insulin degludec: bio-equivalence and similar pharmacodynamics shown for two different formulations (U100 and U200)", DIABETOLOGIA, vol. 54, no. Suppl. 1, September 2011 (2011-09), page S427, XP002723771, & 47TH ANNUAL MEETING OF THE EUROPEAN-ASSOCIATION-FOR-THE-STUDY-OF-DIAB ETES (EASD); LISBON, PORTUGAL; SEPTEMBER 12 -16, 2011

## Description

### FIELD OF THE INVENTION

The present invention relates to a novel use of and dosing regimen for long acting insulin analogues, which is useful in the treatment of diabetes and hyperglycaemia. Furthermore, the dosing regimen of the present invention has been surprising found to lead to improved reductions in blood glucose levels while reducing the risk of hypoglycaemic events, especially in patients requiring high doses of insulin.

### BACKGROUND TO THE INVENTION

Basal insulins are an important treatment option in people with type 2 diabetes (T2DM), with progressively higher doses of insulin required over the duration of the disease. Moreover, approximately 90% of people with T2DM in the US are overweight; thus contributing to an increased need for larger insulin doses than a patient of normal weight.¹ Treatment guidelines currently provide insulin as an early option in the patient-centred approach to the treatment of T2DM.² However, insulin initiation is still often delayed due to both clinician factors (e.g., clinical inertia) and patient factors (e.g., fear of hypoglycaemia and misconceptions about insulin). Insulin treatment intensification can be associated with an increase in hypoglycaemic events.

Obese patients are often less sensitive to exogenous insulin and, therefore, require higher doses to maintain good glycaemic control. Approximately 30% of patients with T2DM using basal insulin require >60 units (U) daily. The use of a highly concentrated formulation of regular human insulin, U-500 regular human insulin (Humulin R® U-500, Eli Lilly and Company, Indianapolis, IN), was originally developed to address high insulin requirements. The frequency of use of U-500 regular human insulin increased dramatically by more than 70% in the US between 2005 and 2008³ primarily in response to the escalating number of people with T2DM and obesity.

Currently marketed insulin pen devices only allow the administration of a maximum of 80 U per injection and administration of larger volumes of insulin has typically required the use of a vial and syringe or the addition of a second injection in order to administer the full dose. Very large doses of insulin delivered as a single injection with a syringe can be painful, cause discomfort at the site of injection, and be physically challenging to smoothly deliver such a large volume.⁴

Hoevelmann et al., Diabetologia (2012) 55:S374-S375 discloses a U200/mL insulin degludec product and its use for glucose lowering.
Wang et al, Diabetes, Metabolic Syndrome and Obesity: Targets and Therapy (2012)5:191-204 discloses insulin degludec as a basal insulin for once daily injection for treating type 1 diabetes and type 2 diabetes.
Korsatko et al, Diabetologia (2011)54:S427 discloses the use of U100 and U200 formulations of insulin degludec and concludes them to be bioequivalent and having similar pharmacodynamic profiles at steady state.

There is a need in the art for a new insulin formulation for the facile treatment of patients requiring high amounts of delivered insulin that addresses the problems identified above.

### SUMMARY OF THE INVENTION

The present invention is based on the surprising finding that long acting insulin analogues when administered to a patient requiring high amounts, such as amounts of greater than 80 U/administration of delivered insulin lead to an improved glucose lowering effect while also reducing the risk of hypoglycaemic events, in particular when administered in high concentration such as concentration of greater than 200 U/mL. Indeed, as illustrated below, when administered to patients requiring high amounts of insulin, the long acting insulin analogues of the invention at high concentration are associated with the benefit of a markedly lower rate of both confirmed and nocturnal hypoglycaemia at a comparable or better glycaemic control in subjects needing high daily insulin doses, especially in comparison with other long acting insulins of the art.

Accordingly, in one aspect, the present invention provides a long acting insulin analogue for use in reducing the risk of hypoglycaemia in a patient suffering from diabetes and requiring amounts of delivered insulin of greater than 80 U/administration, wherein the long acting insulin analogue is administered once daily as a single injection to said patient in an amount of greater than 80 U/administration and in the form of a pharmaceutical composition comprising said insulin at a concentration of 200 U/mL or greater,
and wherein the long acting insulin analogue is a naturally occurring insulin or an insulin analogue having a side chain attached either to the α-amino group of the N-terminal amino acid residue of the B chain or to the ε-amino group of a Lys residue present in the B chain of the parent insulin, the side chain being of the general formula (I):

-W-X-Y-Z

wherein W is:
- an α-amino acid residue having a carboxylic acid group in the side chain which residue forms, with one of its carboxylic acid groups, an amide group together with the α-amino group of the N-terminal amino acid residue of the B chain or together with the ε-amino group of a Lys residue present in the B chain of the parent insulin;
- a chain composed of two, three or four α-amino acid residues linked together via amide bonds, which chain - via an amide bond - is linked to the α-amino group of the N-terminal amino acid residue of the B chain or to the ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain; or
- a covalent bond from X to the α-amino group of the N-terminal amino acid residue of the B chain or to the ε-amino group of a Lys residue present in the B chain of the parent insulin;
wherein X is:
- -CO-;
- -COCH(COOH)CO-;
- -CON(CH₂COOH)CH₂CO-;
- -CON(CH₂COOH)CH₂CON(CH₂COOH)CH₂CO-;
- -CON(CH₂CH₂COOH)CH₂CH₂CO-;
- -CON(CH₂CH₂COOH)CH₂CH₂CON(CH₂CH₂COOH)CH₂CH₂CO-;
- -CONHCH(COOH)(CH₂)₄NHCO-;
- -CON(CH₂CH₂COOH)CH₂CO-; or
- -CON(CH₂COOH)CH₂CH₂CO-.
that
a) when W is an amino acid residue or a chain of amino acid residues, *via* a bond from the underscored carbonyl carbon forms an amide bond with an amino group in W, or
b) when W is a covalent bond, *via* a bond from the underscored carbonyl carbon forms an amide bond with the N-terminal α-amino group in the B chain or with the ε-amino group of a Lys residue present in the B chain of the parent insulin;
wherein Y is:
• -(CH₂)ₘ- where m is an integer in the range of 6 to 32;
• - a divalent hydrocarbon chain comprising 1, 2 or 3 -CH=CH- groups and a number of -CH₂- groups sufficient to give a total number of carbon atoms in the chain in the range of 10 to 32;
• - a divalent hydrocarbon chain of the formula -(CH₂)ᵥC₆H₄(CH₂)_{W} - wherein v and w are integers or one of them is zero so that the sum of v and w is in the range of 6 to 30; and
wherein Z is:
- -COOH;
- -CO-Asp;
- -CO-Glu;
- -CO-Gly;
- -CO-Sar;
- -CH(COOH)₂;
- -N(CH₂COOH)₂;
- -SO₃H; or
- -PO₃H;
and any Zn²⁺ complexes thereof, provided that when W is a covalent bond and X is -CO-, then Z is different from -COOH.
The long acting insulin analogue can be administered to the patient by himself/herself - such as via a suitable device. In addition, or in the alternative, in each instance, the long acting insulin analogue can be administered to the patient by someone else - such as via a suitable device.

Administration may be via any suitable means known to the skilled person, in particular by injection. Accordingly, preferred aspects of the invention are as follows.

The long acting insulin analogue may have an insulin action (e.g. keep blood sugar levels at a steady and stable level) for up to at least 24 hours. As used herein, "for up to at least 24 hours" actually means "for at least 24 hours", from administration.

In one embodiment the long acting insulin analogue may have an insulin action (e.g. keep blood sugar levels at a steady and stable level) for up to at least 30 hours. As used herein, "for up to at least 30 hours" actually means "for at least 30 hours" from administration.

In another embodiment the long acting insulin analogue may have an insulin action, e.g. keep blood sugar levels at a steady and stable level, for up to at least 36 hours. As used herein, "for up to at least 36 hours" actually means "for at least 36 hours" from administration.

In another embodiment the long acting insulin analogue may have an insulin action, e.g. keep blood sugar levels at a steady and stable level, for up to at least 42 hours. As used herein, "for up to at least 42 hours" actually means "for at least 42 hours" from administration.

In another embodiment the long acting insulin analogue may have an insulin action, e.g. keep blood sugar levels at a steady and stable level, for up to at least 48 hours. As used herein, "for up to at least 48 hours" actually means "for at least 48 hours" from administration.

### DESCRIPTION OF THE DRAWINGS

**Figure 1** shows the change in HbA_{1c} levels over time relative to baseline according to dose following administration of IDeg U-200 and IGlar U-100.
**Figure 2** shows the change in HbA_{1c} levels over time according to dose following administration of IDeg U-200 and IGlar U-100.
**Figure 3** shows the change in FPG levels over time following administration of IDeg U-200 and IGlar U-100.
**Figure 4** shows the change in HbA_{1c} levels over time relative to baseline according to dose following administration of IDeg U-200 and IDeg U-100.
**Figure 5** shows the change in HbA_{1c} levels over time according to dose following administration of IDeg U-200 and IDeg U-100.

### DETAILED DESCRIPTION OF THE INVENTION

As mentioned above the present invention provides long-acting insulin analogues for use in one or more of:
- treating a patient suffering from diabetes wherein said patient requires high amounts of delivered insulin;
- reducing the risk of hypoglycaemia in a patient suffering from diabetes wherein said patient requires high amounts of delivered insulin.

### Insulin Analogue

The term "human insulin" as used herein means the human insulin hormone whose structure and properties are well-known. Human insulin has two polypeptide chains, named the A-chain and the B-chain. The A-chain is a 21 amino acid peptide and the B-chain is a 30 amino acid peptide, the two chains being connected by disulphide bridges: a first bridge between the cysteine in position 7 of the A-chain and the cysteine in position 7 of the B-chain, and a second bridge between the cysteine in position 20 of the A-chain and the cysteine in position 19 of the B-chain. A third bridge is present between the cysteines in position 6 and 11 of the A-chain.

The term "insulin analogue" as used herein means a modified human insulin wherein one or more amino acid residues of the insulin have been exchanged for another amino acid residue and/or wherein one or more amino acid residues have been deleted from the insulin and/or wherein one or more amino acid residues have been added and/or inserted to the insulin.

In one embodiment an insulin analogue comprises less than 10 amino acid modifications (substitutions, deletions, additions (including insertions) and any combination thereof) relative to human insulin, alternatively less than 9, 8, 7, 6, 5, 4, 3, 2 or 1 modification relative to human insulin.

Modifications in the insulin molecule are denoted stating the chain (A or B), the position, and the one or three letter code for the amino acid residue substituting the native amino acid residue.

By "desB30" or "B(1-29)" is meant a natural insulin B chain or an analogue thereof lacking the B30 amino acid and "A(1-21)" means the natural insulin A chain. Thus, e.g., A21Gly,B28Asp,desB30 human insulin is an analogue of human insulin where the amino acid in position 21 in the A chain is substituted with glycine, the amino acid in position 28 in the B chain is substituted with aspartic acid, and the amino acid in position 30 in the B chain is deleted.

Herein terms like "A1", "A2" and "A3" etc. indicates the amino acid in position 1, 2 and 3 etc., respectively, in the A chain of insulin (counted from the N-terminal end). Similarly, terms like B1, B2 and B3 etc. indicates the amino acid in position 1, 2 and 3 etc., respectively, in the B chain of insulin (counted from the N-terminal end). Using the one letter codes for amino acids, terms like A21A, A21G and A21Q designates that the amino acid in the A21 position is A, G and Q, respectively. Using the three letter codes for amino acids, the corresponding expressions are A21Ala, A21Gly and A21Gln, respectively.

Herein the terms "A(0)" or "B(0)" indicate the positions of the amino acids N-terminally to A1 or B1, respectively. The terms A(-1) or B(-1) indicate the positions of the first amino acids N-terminally to A(0) or B(0), respectively. Thus A(-2) and B(-2) indicate positions of the amino acids N-terminally to A(-1) and B(-1), respectively, A(-3) and B(-3) indicate positions of the amino acids N-terminally to A(-2) and B(-2), respectively, and so forth. The terms A22 or B31 indicate the positions of the amino acids C-terminally to A21 or B30, respectively. The terms A23 or B32 indicate the positions of the first amino acids C-terminally to A22 or B31, respectively. Thus A24 and B33 indicate positions of the amino acids C-terminally to A23 and B32, respectively, and so forth.

Herein, the term "amino acid residue" is an amino acid from which, formally, a hydroxy group has been removed from a carboxy group and/or from which, formally, a hydrogen atom has been removed from an amino group.

One example of insulin analogues is desB30 human insulin.

The term "insulin analogue" also refers to modified human insulin wherein in addition to, or instead of the modifications described above, there may be a substituent, also called side-chain, bonded (synonym for "attached"), in particular covalently bonded, to an amino acid residue, in particular to any available position on an amino acid residue. In such case, the human insulin to which a substituent is attached is called "parent insulin".
Suitable substituents are, for example, amides, carbohydrates, alkyl groups, acyl groups, esters, PEGylations, and the like.

Thus further examples of insulin analogues according to the invention include LysB29(Nε-hexadecandioyl-γ-Glu) des(B30) human insulin.

To effect covalent attachment of the substituents to the optionally modified human insulin, groups, e.g. the end groups, of the substituents are provided in activated form, i.e. with reactive functional groups.

Hence, the insulin analogue of the present invention can include a naturally occurring insulin or an insulin analogue modified as described above (e.g. wherein one or more amino acid residues of the insulin have been exchanged for another amino acid residue and/or wherein one or more amino acid residues have been deleted from the insulin and/or wherein one or more amino acid residues have been added and/or inserted to the insulin) which optionally has a substituent in one or more positions along the insulin backbone.

In a preferred embodiment of the invention the insulin analogue is a long acting insulin analogue.

### Long Acting Insulin Analogue

The term "long acting insulin analogue(s)" means that the insulin analogue(s), as described above, have an insulin action (e.g. keep blood sugar levels at a steady and stable level) for at least up to 24 hours, preferably up to 30 hours, more preferably up to 36 hours, more preferably still up to 42 hours, more preferably still up to 48 hours. The expression "at least up to 24 hours" means "at least 24 hours" from administration, i.e. "24 hours or longer than 24 hours after administration".

A 'long-acting' insulin analogue may have an insulin action that:
(a) exhibits in physiological conditions, at least in part, the insulin receptor binding of the naturally occurring insulin, preferably, at least 0.01% of the insulin receptor binding of the naturally occurring insulin, for example, at least 0.1%, at least, 1%, at least 5%, at least 10%, at least 15%, at least 20%, at least 25% at least 50%, at least 65%, at least 75%, at least 85%, at least 95%, at least 100%, at least 110%, at least 120%, at least 130%, at least 140% or at least 150% of the insulin receptor binding of the naturally occurring insulin, and/or, at least in part, the potency of the naturally occurring insulin, preferably, at least 25% of the potency of the naturally occurring insulin, for example, at least 50%, at least 65%, at least 75%, at least 85%, at least 95%, at least 100%, at least 110%, at least 120%, at least 130%, at least 140% or at least 150% of the potency of the naturally occurring insulin;
(b) exhibits a mean terminal half life of at least 18 hr in physiological conditions when injected subcutaneously, for example, greater than 18 hours, at least 20 hours, greater than 20 hours, greater than 22 hours, at least 22.5 hours, or greater than 24 hours, at least 25 hours, at least 27.5 hours, at least 30 hours, at least 32.5, at least 35 hours, at least 37.5 hours, or at least 40 hours, or between 18 and 40 hours, between 20 and 40 hours, between 24 and 40 hours.
Preferably, the 'long acting" insulin analogue may have an insulin action that also:
(c) induces in a subject a maximum deviation from mean insulin concentration (AUCF%) over a 24 hour period of ≤ ± 20, for example, ≤ ± 18, ≤ ± 17, ≤ ± 16, ≤ ± 15, ≤ ± 14, ≤ ± 13, ≤ ± 12, ≤ ± 11, ≤ ± 10, ≤ ± 9, ≤ ± 8, ≤ ± 7, ≤ ± 6, ≤ ± 5, ≤ ± 4, ≤ ± 3, ≤ ± 2, ≤ ± 1, ≤ ± 0.5, ≤ ± 0.1.
Insulin receptor binding may be determined by any suitable means known in the art. However preferably, insulin receptor binding is determined using the method provided in the foregoing examples (assay (I) - Insulin receptor binding, as defined in WO2005/012347).
Insulin potency may be determined by any suitable means known in the art. However preferably, insulin potency is determined using the method provided in the foregoing examples (assay (II)-Potency, as defined in WO2005/012347).
Insulin mean terminal half life may be determined by any suitable means known in the art, for example, see Heise T, Nosek L, Bøttcher SG, Hastrup H, Haahr H, 2012, 'Ultra-long-acting insulin degludec has a flat and stable glucose-lowering effect in type 2 diabetes' Diabetes Obes Metab., 14(10):944-50, the disclosures of which are referenced herein. Insulin mean terminal half-life may alternatively be determined using the method provided in WO2005/012347 (assay III).
Maximum deviation from mean insulin concentration (AUCF%) may be determined by any suitable means known in the art (see, for example, Heise et al., Poster EASD 2011; or Heise et al., Poster ADA 2011 entitled "Insulin degludec has a two-fold longer half-life and a more consistent pharmacokinetic profile that insulin glargine" Diabetes 2011: 60(Suppl 1):LB11 (Abstract 37-LB); or Heise et al., 2012, Diabetes, Obesity and Metabolism, 14(10):944-50). The long acting insulin analogue is any one or more of the compounds disclosed in WO 2005/012347. In some instances, these compounds are referred as being "the '347 derivatives". Hence, preferably the long acting insulin analogue is a '347 derivative, i.e. a derivative of a naturally occurring insulin or of an insulin analogue having a side chain attached either to the α-amino group of the N-terminal amino acid residue of the B chain or to the ε-amino group of a Lys residue present in the B chain of the parent insulin, the side chain being of the general formula (I):

-W-X-Y-Z

wherein W is:
- an α-amino acid residue having a carboxylic acid group in the side chain which residue forms, with one of its carboxylic acid groups, an amide group together with the α-amino group of the N-terminal amino acid residue of the B chain or together with the ε-amino group of a Lys residue present in the B chain of the parent insulin;
- a chain composed of two, three or four α-amino acid residues linked together via amide bonds, which chain - via an amide bond - is linked to the α-amino group of the N-terminal amino acid residue of the B chain or to the ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain; or
- a covalent bond from X to the α-amino group of the N-terminal amino acid residue of the B chain or to the ε-amino group of a Lys residue present in the B chain of the parent insulin;
wherein X is:
- -CO-;
- -COCH(COOH)CO-;
- -CON(CH₂COOH)CH₂CO-;
- -CON(CH₂COOH)CH₂CON(CH₂COOH)CH₂CO-;
- -CON(CH₂CH₂COOH)CH₂CH₂CO-;
- -CON(CH₂CH₂COOH)CH₂CH₂CON(CH₂CH₂COOH)CH₂CH₂CO-;
- -CONHCH(COOH)(CH₂)₄NHCO-;
- -CON(CH₂CH₂COOH)CH₂CO-; or
- -CON(CH₂COOH)CH₂CH₂CO-.
that
a) when W is an amino acid residue or a chain of amino acid residues, *via* a bond from the underscored carbonyl carbon forms an amide bond with an amino group in W, or
b) when W is a covalent bond, *via* a bond from the underscored carbonyl carbon forms an amide bond with the N-terminal α-amino group in the B chain or with the ε-amino group of a Lys residue present in the B chain of the parent insulin;
wherein Y is:
• -(CH₂)ₘ- where m is an integer in the range of 6 to 32;
• - a divalent hydrocarbon chain comprising 1, 2 or 3 -CH=CH- groups and a number of -CH₂- groups sufficient to give a total number of carbon atoms in the chain in the range of 10 to 32;
• - a divalent hydrocarbon chain of the formula -(CH₂)ᵥC₆H₄(CH₂)_{W}- wherein v and w are integers or one of them is zero so that the sum of v and w is in the range of 6 to 30; and
wherein Z is:
- -COOH;
- -CO-Asp;
- -CO-Glu;
- -CO-Gly;
- -CO-Sar;
- -CH(COOH)₂;
- -N(CH₂COOH)₂;
- -SO₃H; or
- -PO₃H;
and any Zn²⁺ complexes thereof, provided that when W is a covalent bond and X is -CO-, then Z is different from -COOH.

In the expression "the long acting insulin analogue is a naturally occurring insulin or an insulin analogue having a side chain attached (...)", it is meant that the side chain is attached either to said "naturally occurring insulin" or to said "insulin analogue". It is also meant that said "naturally occurring insulin" or said "insulin analogue" is the "parent insulin" to which the side chain is attached.

In one embodiment the side chain -W-X-Y-Z is attached to the α-amino group of the N-terminal amino acid residue of the B chain of the parent insulin.

In another embodiment of the invention, side chain -W-X-Y-Z is attached to the ε-amino group of a Lys residue present in the B chain of the parent insulin.

In one more specific aspect of this embodiment, the side chain -W-X-Y-Z is attached to the ε-amino group of a Lys residue present in position 28 of the B chain.

In a further more specific aspect of this embodiment, the side chain -W-X-Y-Z is attached to the ε-amino group of a Lys residue present in position 29 of the B chain.

In a further more specific aspect of this embodiment, the side chain -W-X-Y-Z is attached to the ε-amino group of a Lys residue present in position 30 of the B chain.

The substructure W of the side chain -W-X-Y-Z can be a covalent bond. Alternatively, W can be a residue of an α-amino acid having a carboxylic acid group in the side chain and comprising a total of from 4 to 10 carbon atoms. Specifically, W can be the residue of an α-amino acid, that can be coded for by the genetic code. Thus, W can, for example, be selected from the group consisting of α-Asp, β-Asp, α-Glu, and γ-Glu. Further options for W are for example α-hGlu and δ-hGlu.

In a further embodiment, W is a chain composed of two α-amino acid residues of which one has from 4 to 10 carbon atoms and a carboxylic acid group in the side chain while the other has from 2 to 11 carbon atoms but no free carboxylic acid group. The α-amino acid residue with no free carboxylic acid group can be a neutral, codable α-amino acid residue. Examples of W according to this embodiment are: α-Asp-Gly; Gly-α-Asp; β-Asp-Gly; Gly-β-Asp; α-Glu-Gly; Gly-α-Glu; γ-Glu-Gly; Gly-γ-Glu; α-hGlu-Gly; Gly-α-hGlu; δ-hGlu-Gly; and Gly-δ-hGlu.

In a further embodiment, W is a chain composed of two α-amino acid residues, independently having from 4 to 10 carbon atoms, and both having a carboxylic acid group in the side chain. One of these α-amino acid residues or both of them can be codable α-amino acid residues. Examples of W according to this embodiment are: α-Asp-α-Asp; α-Asp-α-Glu; α-Asp-α-hGlu; α-Asp-β-Asp; α-Asp-γ-Glu; α-Asp-δ-hGlu; β-Asp-α-Asp; β-Asp-α-Glu; β-Asp-α-hGlu; β-Asp-β-Asp; β-Asp-γ-Glu; β-Asp-δ-hGlu; α-Glu-α-Asp; α-Glu-α-Glu; α-Glu-α-hGlu; α-Glu-β-Asp; α-Glu-γ-Glu; α-Glu-δ-hGlu; γ-Glu-α-Asp; γ-Glu-α-Glu; γ-Glu-α-hGlu; γ-Glu-β-Asp; γ-Glu-γ-Glu; γ-Glu-δ-hGlu; α-hGlu-α-Asp; α-hGlu-α-Glu; α-hGlu-α-hGlu; α-hGlu-β-Asp; α-hGlu-γ-Glu; α-hGlu-δ-hGlu; δ-hGlu-α-Asp; δ-hGlu-α-Glu; δ-hGlu-α-hGlu; δ-hGlu-β-Asp; δ-hGlu-γ-Glu; and δ-hGlu-δ-hGlu.

In a further embodiment, W is a chain composed of three α-amino acid residues, independently having from 4 to 10 carbon atoms, the amino acid residues of the chain being selected from the group of residues having a neutral side chain and residues having a carboxylic acid group in the side chain so that the chain has at least one residue which has a carboxylic acid group in the side chain. In one embodiment, the amino acid residues are codable residues.

In a further embodiment, W is a chain composed of four α-amino acid residues, independently having from 4 to 10 carbon atoms, the amino acid residues of the chain being selected from the group having a neutral side chain and residues having a carboxylic acid group in the side chain so that the chain has at least one residue which has a carboxylic acid group in the side chain. In one embodiment, the amino acid residues are codable residues.

In one embodiment W can be connected to the ε-amino group of the Lys residue in the B-chain via an urea derivative.

The substructure X of the side chain -W-X-Y-Z can be a group of the formula -CO- that, via a bond from the underscored carbonyl carbon, forms an amide bond with an amino group in W or, when W is a covalent bond, with the N-terminal α-amino group in the B chain or with the ε-amino group of a Lys residue present in the B chain of the parent insulin.

The substructure Y of the side chain -W-X-Y-Z can be a group of the formula -(CH₂)ₘ- where m is an integer in the range of from 6 to 32, from 8 to 20, from 12 to 20, or from 12-16.

In another embodiment, Y is a divalent hydrocarbon chain comprising 1, 2 or 3 -CH=CH-groups and a number of -CH₂- groups sufficient to give a total number of carbon atoms in the chain in the range of from 6 to 32, from 10 to 32, from 12 to 20, or from 12-16.

In another embodiment, Y is a divalent hydrocarbon chain of the formula -(CH₂)ᵥC₆H₄(CH₂)_{W} - wherein v and w are integers or one of them is zero so that the sum of v and w is in the range of from 6 to 30, from 10 to 20, or from 12-16.

In one embodiment, the substructure Z of the side chain -W-X-Y-Z is -COOH provided that when W is a covalent bond and X is -CO-, then Z is different from -COOH. In one embodiment Z is -COOH. In another embodiment, Z is -CO-Asp. In another embodiment, Z is -CO-Glu. In another embodiment, Z is -CO-Gly. In another embodiment, Z is -CO-Sar. In another embodiment, Z is -CH(COOH)₂. In another embodiment, Z is -N(CH₂COOH)₂. In another embodiment, Z is -SO₃H. In another embodiment, Z is -PO₃H.

In a further embodiment W is selected from the group consisting of α-Asp, β-Asp, α-Glu, and γ-Glu; X is -CO- or -COCH(COOH)CO; Y is -(CH₂)ₘ- where m is an integer in the range of 12-18 and Z is -COOH or -CH(COOH)₂.

The insulin moiety - in the present text also referred to as the parent insulin - of a '347 derivative can be a naturally occurring insulin such as human insulin or porcine insulin. Alternatively, the parent insulin can be an insulin analogue.

In one group of parent insulin analogues, the amino acid residue at position A21 is Asn.

In another group of parent insulin analogues, the amino acid residue at position A21 is Gly. Specific examples from this group of analogues are Gly^{A21} human insulin, Gly^{A21} des(B30)human insulin; and Gly^{A21}Arg^{B31}Arg^{B32} human insulin.

In another group of parent insulin analogues, the amino acid residue at position B1 has been deleted. A specific example from this group of parent insulin analogues is des(B1) human insulin.

In another group of parent insulin analogues, the amino acid residue at position B30 has been deleted. A specific example from this group of parent insulin analogues is des(B30) human insulin.

In another group of parent insulin analogues, the amino acid residue at position B28 is Asp. A specific example from this group of parent insulin analogues is Asp^{B28} human insulin) or otherwise stated as AspB28).

In another group of parent insulin analogues, the amino acid residue at position B28 is Lys and the amino acid residue at position B29 is Pro. A specific example from this group of parent insulin analogues is Lys^{B28}Pro^{B29} human insulin.

In another group of parent insulin analogues the amino acid residue in position B30 is Lys and the amino acid residue in position B29 is any codable amino acid except Cys, Met, Arg and Lys. An example is an insulin analogue where the amino acid residue at position B29 is Thr and the amino acid residue at position B30 is Lys. A specific example from this group of parent insulin analogues is Thr^{B29}Lys^{B30} human insulin.

In another group of parent insulin analogues, the amino acid residue at position B3 is Lys and the amino acid residue at position B29 is Glu. A specific example from this group of parent insulin analogues is Lys^{B3}Glu^{B29} human insulin.

In one embodiment the parent insulin is selected from the group consisting of human insulin; des(B1) human insulin; des(B30) human insulin; GlyA21 human insulin; GlyA21 des(B30)human insulin; AspB28 human insulin; porcine insulin; LysB28ProB29 human insulin; GlyA21ArgB31ArgB32 human insulin; and LysB3GluB29 human insulin.

Examples of '347 derivatives, i.e. of insulin of definition A, useful in the invention are the following compounds:
N^{εB29}-(N^{α}-(HOOC(CH₂)₁₄CO)-γ-Glu) des(B30) human insulin;
N^{εB29}-(N^{α}-(HOOC(CH₂)₁₅CO)-γ-Glu) des(B30) human insulin;
N^{εB29}-(N^{α}-(HOOC(CH₂)₁₆CO)-γ-Glu) des(B30) human insulin;
N^{εB29}-(N^{α}-(HOOC(CH₂)₁₇CO)-γ-Glu) des(B30) human insulin;
N^{εB29}-(N^{α}-(HOOC(CH₂)₁₈CO)-γ-Glu) des(B30) human insulin;
N^{εB29}-(N^{α}-(HOOC(CH₂)₁₆CO)-γ-Glu-N-(γ-Glu)) des(B30) human insulin;
N^{εB29}-(N^{α}-(Asp-OC(CH₂)₁₆CO)-γ-Glu) des(B30) human insulin;
N^{εB29}-(N^{α}-(Glu-OC(CH₂)₁₄CO)-γ-Glu) des(B30) human insulin;
N^{εB29}-(N^{α}-(Glu-OC(CH₂)₁₄CO-) des(B30) human insulin;
N^{εB29}-(N^{α}-(Asp-OC(CH₂)₁₆CO-) des(B30) human insulin;
N^{εB29}-(N^{α}-(HOOC(CH₂)₁₆CO)-α-Glu-N-(β-Asp)) des(B30) human insulin;
N^{εB29}-(N^{α}-(Gly-OC(CH₂)₁₃CO)-γ-GLu) des(B30) human insulin;
N^{εB29}-(N^{α}-(Sar-OC(CH₂)₁₃CO)-γ-Glu) des(B30) human insulin;
N^{εB29}-(N^{α}-(HOOC(CH₂)₁₃CO)-γ-Glu) des(B30) human insulin;
(N^{εB29}-(N^{α}-(HOOC(CH₂)₁₃CO)-β-Asp) des(B30) human insulin;
N^{εB29}-(N^{α}-(HOOC(CH₂)₁₃CO)-α-Glu) des(B30) human insulin;
N^{εB29}-(N^{α}-(HOOC(CH₂)₁₆CO)-γ-D-Glu) des(B30) human insulin;
N^{εB29}-(N^{α}-(HOOC(CH₂)₁₄CO)-β-D-Asp) des(B30) human insulin;
N^{εB29}-(N-HOOC(CH₂)₁₆CO-β-D-Asp) des(B30) human insulin;
N^{εB29}-(N-HOOC(CH₂)₁₄CO-IDA) des(B30) human insulin;
N^{εB29}-[N-(HOOC(CH₂)₁₆CO)-N-(carboxyethyl)-Gly] des(B30) human insulin;
N^{εB29}-[N-(HOOC(CH₂)₁₄CO)-N-(carboxyethyl)-Gly] des(B30) human insulin; and
N^{εB29}-[N-(HOOC(CH₂)₁₄CO)-N-(carboxymethyl)-β-Ala] des(B30) human insulin.

In one embodiment the long acting insulin analogue is N^{εB29}-(N^{α}-(HOOC(CH₂)₁₄CO)-γ-Glu) des(B30) human insulin.

In one embodiment the long acting insulin analogue is N^{εB29}-(N^{α}-(HOOC(CH₂)₁₅CO)-γ-Glu) des(B30) human insulin.

In one embodiment the long acting insulin analogue is N^{εB29}-(N^{α}-(HOOC(CH₂)₁₆CO)-γ-Glu) des(B30) human insulin.

In one embodiment the long acting insulin analogue is N^{εB29}-(N^{α}-(HOOC(CH₂)₁₇CO)-γ-Glu) des(B30) human insulin.

In one embodiment the long acting insulin analogue is N^{εB29}-(N^{α}-(HOOC(CH₂)₁₈CO)-γ-Glu) des(B30) human insulin.

In one embodiment the long acting insulin analogue is N^{εB29}-(N^{α}-(HOOC(CH₂)₁₆CO)-γ-Glu-N-(γ-Glu)) des(B30) human insulin.

In one embodiment the long acting insulin analogue is N^{εB29}-(N^{α}-(Asp-OC(CH₂)₁₆CO)-γ-Glu) des(B30) human insulin.

In one embodiment the long acting insulin analogue is N^{εB29}-(N^{α}-(Glu-OC(CH₂)₁₄CO)-γ-Glu) des(B30) human insulin.

In one embodiment the long acting insulin analogue is N^{εB29}-(N^{α}-(Glu-OC(CH₂)₁₄CO-) des(B30) human insulin.

In one embodiment the long acting insulin analogue is N^{εB29}-(N^{α}-(Asp-OC(CH₂)₁₆CO-) des(B30) human insulin.

In one embodiment the long acting insulin analogue is N^{εB29}-(N^{α}-(HOOC(CH₂)₁₆CO)-α-Glu-N-(β-Asp)) des(B30) human insulin.

In one embodiment the long acting insulin analogue is N^{εB29}-(N^{α}-(Gly-OC(CH₂)₁₃CO)-γ-Glu) des(B30) human insulin.

In one embodiment the long acting insulin analogue is N^{εB29}-(N^{α}-(Sar-OC(CH₂)₁₃CO)-γ-Glu) des(B30) human insulin.

In one embodiment the long acting insulin analogue is N^{εB29}-(N^{α}-(HOOC(CH₂)₁₃CO)-γ-Glu) des(B30) human insulin.

In one embodiment the long acting insulin analogue is (N^{εB29}-(N^{α}-(HOOC(CH₂)₁₃CO)-β-Asp) des(B30) human insulin.

In one embodiment the long acting insulin analogue is N^{εB29}-(N^{α}-(HOOC(CH₂)₁₃CO)-α-Glu) des(B30) human insulin.

In one embodiment the long acting insulin analogue is N^{εB29}-(N^{α}-(HOOC(CH₂)₁₆CO)-γ-D-Glu) des(B30) human insulin.

In one embodiment the long acting insulin analogue is N^{εB29}-(N^{α}-(HOOC(CH₂)₁₄CO)-β-D-Asp) des(B30) human insulin.

In one embodiment the long acting insulin analogue is N^{εB29}-(N^{α}-(HOOC(CH₂)₁₄CO)-β-D-Asp) des(B30) human insulin.

In one embodiment the long acting insulin analogue is N^{εB29}-(N-HOOC(CH₂)₁₆CO-β-D-Asp) des(B30) human insulin.

In one embodiment the long acting insulin analogue is N^{εB29}-(N-HOOC(CH₂)₁₄CO-IDA) des(B30) human insulin.

In one embodiment the long acting insulin analogue is N^{εB29}-[N-(HOOC(CH₂)₁₆CO)-N-(carboxyethyl)-Gly] des(B30) human insulin.

In one embodiment the long acting insulin analogue is N^{εB29}-[N-(HOOC(CH₂)₁₄CO)-N-(carboxyethyl)-Gly] des(B30) human insulin.

In one embodiment the long acting insulin analogue is N^{εB29}-[N-(HOOC(CH₂)₁₄CO)-N-(carboxymethyl)-β-Ala] des(B30) human insulin.

When zinc complexes of a '347 derivative are provided, above four Zn²⁺ ions, five Zn²⁺ ions six Zn²⁺ ions or up to 12 Zn²⁺ ions will be present per 6 molecules of the '347 derivative. In one embodiment the insulin derivative is in the form of a zinc complex, wherein 6 molecules of the '347 derivative binds two zinc ions, three zinc ions, four zinc ions, five zinc ions, six zinc ions, seven zinc ions, eight zinc ions, nine zinc ions or ten zinc ions. Solutions of zinc complexes of the insulin derivatives will contain mixtures of such species.
Details pertaining to the preparation, formulation, pharmacology and other characteristics of relevance for the '347 derivatives are set forth in WO 2005/012347.
The present invention may comprise one or more long acting insulin analogues.
The one or more long acting insulin analogue(s) may be any one or more of the long acting insulin analogues presented above.
In one preferred embodiment the long acting insulin analogue is selected from insulin degludec, (also known as NεB29-(Nα-(HOOC(CH2)14CO)-γ-Glu)des(B30)-human insulin, or N^{εB29}-ω-carboxy-pentadecanoyl-γ-L-glutamylamide desB30 human insulin, or IDeg).
In a particularly preferred embodiment the long acting insulin of the present invention is insulin degludec (IDeg). Insulin degludec (IDeg) is a new generation, long-acting basal insulin in clinical development with a duration of action greater than 42 hours.
Accordingly, preferred aspects of the invention are as follows.
In one aspect, the present invention provides a long acting insulin analogue for use in treating a patient suffering from diabetes and requiring amounts of delivered insulin of greater than 80 U/administration, wherein the long acting insulin analogue is administered to said patient by injection in an amount of greater than 80 U/injection and in the form of a pharmaceutical composition comprising said insulin at a concentration of 200 U/ml or greater, wherein the long acting insulin analogue is insulin degludec (IDeg). In a preferred embodiment, the long acting insulin analogue is administered in the form of a pharmaceutical composition comprising said insulin at a concentration of 200 U/mL.
In a further aspect the present invention provides a long acting insulin analogue for use in providing beneficial glycaemic control in a patient suffering from diabetes and requiring amounts of delivered insulin of greater than 80 U/administration, wherein the long acting insulin analogue is administered to said patient by injection in an amount of greater than 80 U/injection and in the form of a pharmaceutical composition comprising said insulin at a concentration 200 U/ml or greater, wherein the long acting insulin analogue is insulin degludec (IDeg). In a preferred embodiment, the long acting insulin analogue is administered in the form of a pharmaceutical composition comprising said insulin at a concentration of 200 U/mL.

In a further aspect, the present invention provides a long acting insulin analogue for use in reducing the risk of hypoglycaemia in a patient suffering from diabetes and requiring amounts of delivered insulin of greater than 80 U/administration, wherein the long acting insulin analogue is administered to said patient once daily by a single injection and in an amount of greater than 80 U/ injection, wherein the long acting insulin analogue is insulin degludec (IDeg) and in the form of a pharmaceutical composition comprising said insulin at a concentration of 200 U/mL or greater.

### Dosing Regimen

The long acting insulin analogues of the present invention are for use in administration to a patient requiring high doses of insulin in an amount of greater than 80 U/administration.

As used herein the term "U" refers to a unit of insulin or of an insulin analogue or derivative. The designation "U" with a number following indicates the concentration as measured by the number of units per ml of fluid volume (Joslin's Diabetes Deskbook, 2nd edition, Chapter 9 Using insulin to treat diabetes - general principles, page 268). Further information about the meaning of "U" can be found in a document from the EMA (reference EMEA/CHMP/BWP/124446/2005) entitled "Guideline on potency labelling for insulin analogue containing products with particular reference to the use of "International Units" or "Units"". One unit of insulin analogue is equivalent to one IU of human insulin. "IU" refers to an international unit of human insulin as defined according to the WHO Expert Committee on Biological Standardization. IU is a standardized parameter. For commercial insulins, the labels indicate the content of 1 U (unit) of the particular insulin analogue. One U of the insulin analogue is calibrated against one IU of human insulin such that there is a 1:1 dosing ratio.

As used herein the term "U/administration" means units of insulin analogue per administration.

As used herein the term "administration" refers to the act of delivering a dose of the long acting insulin analogue to said patient. Preferably administration is by injection. More preferably, administration is by subcutaneous injection.

Accordingly, in one embodiment the long acting insulin analogues of the present invention are for use in administration once daily by a single injection to a patient requiring high doses of insulin in an amount of greater than 80 U/injection in the form of a pharmaceutical composition comprising said insulin at a concentration of 200 U/mL or greater.

In an embodiment the long acting insulin analogues of the present invention are administered once daily by a single injection, in an amount of between 80 U/administration and 160 U/administration, e.g. >80U and ≤160 U/administration.

Preferably the long acting insulin analogues of the present invention are administered using pen injection devices.

Pen injection devices are more convenient and easier to transport than traditional vial and syringe devices. Furthermore, they are easier to use and provide more accurate dosing.

In a preferred embodiment the pen injection device is a FlexTouch™ pen device marketed by the present Applicant.

The long acting analogues for use according to the present invention are administered to patients requiring high doses of delivered insulin. In one embodiment the patient of the present invention requires high doses of basal insulin.

A patient requiring "high amounts" of delivered insulin or "high doses" of insulin as used herein refers to a patient requiring more than 80U of insulin per administration. In one embodiment, it refers to a patient between 80U and 160U per administration.

In one embodiment high amounts are amounts greater than 80 U/day of delivered insulin.

In one embodiment the long acting insulin analogues for use according to the present invention are administered to a patient requiring greater than 80 U/day of delivered insulin in an amount of greater than 80 U/administration.

In one embodiment the patient of the present invention is obese. In another embodiment the patient of the present invention has a Body/Mass Index (BMI) of 30 or more. In another embodiment the patient of the present invention has a Body/Mass Index (BMI) of between 30 and 45 kg/m².

Body Mass Index (BMI) is a simple index of weight-for-height that is commonly used to classify underweight, overweight and obesity in adults. It is defined as the weight in kilograms divided by the square of the height in metres (kg/m²). BMI is a well known parameter.

Accordingly, in one embodiment of the invention the long acting insulin analogues for use according to the present invention are administered to patient with a BMI of between 30 and 45 kg/m² and in an amount of greater than 80 U/administration.

In one embodiment of the present invention the patient of the present invention is insulin naïve, i.e. has not previously been treated with delivered insulin.

In another embodiment of the present invention the patient of the present invention has previously been treated with basal-only insulin.

In another embodiment the patient of the present invention has previously been treated with oral antidiabetic drugs (OADs).

In another embodiment, the patient of the present invention is undergoing concomitant therapy with oral antidiabetic drugs (OADs).

In a preferred embodiment the OADs are selected from metformin, dipeptidyl peptidase-IV inhibitors, sulfonylurea, glinides, alpha-glucosidase inhibitors, thiazoledinediones, sodium glucose co-transporter 2 (SGLT2) receptor inhibitors and combinations thereof.

In one embodiment the OADs are selected from metformin, dipeptidyl peptidase-IV inhibitors and combinations thereof.

Preferred dipeptidyl peptidase-IV inhibitors are selected from sitagliptin (such as Januvia™ marketed by Merck & Co), vildagliptin (such as Galvus™ marketed in the EU by Novartis), such as saxagliptin (such as Onglyza™ marketed by BMS and AstraZeneca) and linagliptin (such as Trajenta™ marketed by Eli Lilly Co and Boehringer Ingelheim).

### Therapeutic Applications

In one embodiment the long acting insulin analogues for use according to the present invention are for use in treating a patient suffering from diabetes.

The term "diabetes" or "diabetes mellitus" includes type 1 diabetes, type 2 diabetes, gestational diabetes (during pregnancy) and other states that cause hyperglycaemia. The term is used for a metabolic disorder in which the pancreas produces insufficient amounts of insulin, or in which the cells of the body fail to respond appropriately to insulin thus preventing cells from absorbing glucose. As a result, glucose builds up in the blood.

Type 1 diabetes, also called insulin-dependent diabetes mellitus (IDDM) and juvenile-onset diabetes, is caused by B-cell destruction, usually leading to absolute insulin deficiency.

Type 2 diabetes, also known as non-insulin-dependent diabetes mellitus (NIDDM) and adult-onset diabetes, is associated with predominant insulin resistance and thus relative insulin deficiency and/or a predominantly insulin secretory defect with insulin resistance.

Preferably the long acting insulin analogues for use in the present invention are for use in the treatment of type 2 diabetes.

Diabetes often requires insulin treatment to establish proper metabolic control, comprising mainly glycaemic control.

In one embodiment the long acting insulin analogues for use according to the present invention are for use in providing beneficial glycaemic control in a patient suffering from diabetes and requiring high amounts of delivered insulin.

In one embodiment beneficial glycaemic control can be determined by measurement of levels of glycated haemoglobin (HbA_{1c}). In one embodiment beneficial glycaemic control is defined as a reduction of at least 0.5% HbA_{1c} relative to baseline HbA_{1c}; preferably a reduction of at least 0.7% relative to baseline HbA_{1c}; more preferably a reduction of at least 0.9% relative to baseline HbA_{1c}; more preferably reduction of at least 1% relative to baseline HbA_{1c}; more preferably a reduction of at least 1.2% relative to baseline HbA_{1c}; most preferably a reduction of 1.3% relative to baseline HbA_{1c}.

Baseline HbA_{1c} is defined as the level HbA_{1c} before treatment according to the present invention.

In another embodiment, beneficial glycaemic control can be determined by a decrease of the levels of HbA_{1c} to, in one embodiment, about 7% or less; in another embodiment to about 6.5% or less.

HbA_{1c} levels are a commonly used parameter in the art. HbA_{1c} levels may be determined as provided in J. O. Jeppsson, U. Kobold, J. Barr, A. Finke, W. Hoelzel, T. Hoshino, K. Miedema, A. Mosca, P. Mauri, R. Paroni, L. Thienpont, M. Umemoto, and C. Weykamp; Approved IFCC reference method for the measurement of HbA1c in human blood; Clin. Chem. Lab. Med. 40 (1):78-89, 2002, the contents of which is incorporated herein by reference.

In one embodiment beneficial glycaemic control can be determined by measurement of levels of fasting plasma glucose (FPG). In one embodiment beneficial glycaemic control is defined as a reduction of FPG to a level of 7 mmol/L, more preferably 6 mmol/L, more preferably 5 mmol/L.

In one embodiment the long acting insulin analogues for use according to the present invention are for use in reducing the risk of hypoglycaemia in a patient suffering from diabetes and requiring high amounts of delivered insulin.

Hypoglycaemia is a condition wherein a diabetes patient experiences a low plasma glucose concentration leading to symptoms such as sweating, palpitations, hunger, restlessness, anxiety, fatigue, irritability, headache, loss of concentration, somnolence, psychiatric disorders, visual disorders, transient sensory defects, transient motor defects, confusion, convulsions, and coma for example.

As used herein, "hypoglycaemia" may be defined as the situation when a diabetes patient has a plasma glucose concentration of below 3.9 mmol/L, or below 3.5 mmol/L, or 3.3 mmol/L, or below 3.1 mmol/L, or below 2.3 mmol/L; preferably below 3.1 mmol/L.

In one embodiment, the risk of hypoglycaemia resulting from insulin therapy can be determined by calculation of an estimated rate of hypoglycaemic episodes per exposure year.

A rate of hypoglycaemic episodes per exposure year can be determined by dividing the number of episodes by the time of exposure to insulin in years. The estimated rate of hypoglycaemic episodes per exposure year can be determined by regression modelling and including any co-variates as appropriate according to standard statistical processes. Such an approach can be used to analyse the number of treatment-emergent confirmed hypoglycaemic episodes using a negative binomial regression model including treatment, antidiabetic therapy at screening, sex and region as fixed factors, and age as covariate.

In one embodiment the risk of hypoglycaemia is reduced to a rate of hypoglycaemic episodes of less than 2 episodes/exposure in a year; preferably less than 1.5 episodes/exposure in a year; preferably less than 1.0 episodes/exposure year; preferably less than 0.5 episodes/exposure in a year.

In one embodiment the long acting insulin analogues for use according to the present invention are for use in reducing the risk of nocturnal hypoglycaemia. As used herein "nocturnal hypoglycaemia" is defined as hypoglycaemic episodes occurring between 00:01 h and 05:59 h (inclusive).

In one embodiment the risk of nocturnal hypoglycaemia is reduced to a rate of hypoglycaemic episodes of less than 1.0 episodes/exposure year; preferably less than 0.7 episodes/exposure in a year; preferably less than 0.5 episodes/exposure year; preferably less than 0.3 episodes/exposure in a year; preferably less than 0.1 episodes/exposure in a year.

### Pharmaceutical compositions

In one aspect of the present invention the long acting insulin analogue for use according to the present invention is provided as a pharmaceutical composition further comprising at least one pharmaceutically acceptable excipient, diluent or carrier.
Administration of pharmaceutical compositions according to the invention may be through several routes of administration, for example, parenteral administration to patients in need of such a treatment.

The pharmaceutical compositions may be administered to a patient in need of such treatment at several sites, for example, administration in the skin, under the skin, in a muscle or in the abdomen. Subcutaneous administration is preferred.
Carriers, diluents and excipients may be selected from one or more of a buffer system, preservative(s), tonicity agent(s), chelating agent(s), stabilizers and surfactants. In one embodiment of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50% w/w water. Likewise, the term "aqueous solution" is defined as a solution comprising at least 50% w/w water, and the term "aqueous suspension" is defined as a suspension comprising at least 50% w/w water.
In one embodiment the compositions of the present invention comprise a high content of zinc atoms as described in WO2007/074133. In one embodiment the compositions of the present invention comprise more than 4 zinc atoms per 6 molecules of long acting insulin analogue, more than 4 and up to 12 zinc atoms per 6 molecules of long acting insulin analogue or between 4.3 and 12 zinc atoms per 6 molecules of long acting insulin analogue.
Parenteral administration may be performed by subcutaneous, intramuscular, intraperitoneal or intravenous injection by means of a syringe, optionally a pen-like syringe. Alternatively, parenteral administration can be performed by means of an infusion pump.
These formulations can be prepared using the conventional techniques of the pharmaceutical industry. For instance, a parenteral formulation can be prepared by a process which involves dissolving and mixing the ingredients as appropriate to give the desired end product. Thus, according to one procedure, a long acting insulin analogue is dissolved in an amount of water which is somewhat less than the final volume of the composition to be prepared. A buffer system, preservative(s), tonicity agent(s), chelating agent(s), stabilizers and/or surfactants is added as required and the pH value of the solution is adjusted - if necessary - using an acid, e.g. hydrochloric acid, or a base, e.g. aqueous sodium hydroxide as needed. Finally, the volume of the solution is adjusted with water to give the desired concentration of the ingredients.

The concentration of the pharmaceutical composition is greater than or equal to 200 U/mL with respect to the long acting insulin analogue. In a preferred embodiment of the present invention the concentration of the pharmaceutical composition is about 200 U/mL with respect to the long acting insulin analogue.

In one embodiment of the invention there is provided a pharmaceutical composition comprising a long acting insulin analogue and at least one pharmaceutically acceptable excipient, carrier or diluent for use in treating a patient suffering from diabetes and requiring amounts of delivered insulin of greater than 80 U/administration wherein the long acting insulin analogue is administered once daily as a single injection to said patient in an amount of greater than 80 U/administration, and wherein the composition is provided at a concentration of 200 U/mL with respect to the long acting insulin analogue.

In one embodiment of the invention there is provided a pharmaceutical composition comprising a long acting insulin analogue and at least one pharmaceutically acceptable excipient, carrier or diluent for use in treating a patient suffering from diabetes and requiring amounts of delivered insulin of greater than 80 U/administration, wherein the long acting insulin analogue is administered to said patient once daily as a single injection and in an amount of greater than 80 U/ injection, and the composition is provided at a concentration of 200 U/mL with respect to the long acting insulin analogue.

In an aspect of the invention there is provided a pharmaceutical composition comprising a long acting insulin analogue and at least one pharmaceutically acceptable excipient, carrier or diluent for use in treating a patient suffering from diabetes and requiring amounts of delivered insulin of greater than 80 U/administration and wherein the long acting insulin analogue is administered to said patient by injection once daily as a single injection in an amount of greater than 80 U/injection, wherein the composition is provided at a concentration of 200 U/mL or greater with respect to the long acting insulin analogue, wherein the long acting insulin analogue is insulin degludec (IDeg).

In an aspect of the invention there is provided a pharmaceutical composition comprising a long acting insulin analogue and at least one pharmaceutically acceptable excipient, carrier or diluent for use in treating a patient suffering from diabetes and requiring amounts of delivered insulin of greater than 80 U/administration and wherein the long acting insulin analogue is administered to said patient once daily as a single injection and in an amount of greater than 80 U/injection, wherein the long acting insulin analogue is degludec (IDeg), and wherein the long acting insulin analogue reduces the risk of hypoglycaemia.

In one embodiment of the invention there is provided a pharmaceutical composition comprising a long acting insulin analogue and at least one pharmaceutically acceptable excipient, carrier or diluent for use in treating a patient suffering from diabetes and requiring amounts of delivered insulin of greater than 80 U/administration and wherein the long acting insulin analogue is administered once daily as a single injection to said patient by injection and in an amount of greater than 80 U/ injection, and the composition is provided at a concentration of about 200 U/mL with respect to the long acting insulin analogue, wherein the long acting insulin analogue is degludec (IDeg), and wherein the long acting insulin analogue reduces the risk of hypoglycaemia.

In an aspect of the invention there is provided a pharmaceutical composition comprising a long acting insulin analogue and at least one pharmaceutically acceptable excipient, carrier or diluent for use in reducing the risk of hypoglycaemia in a patient suffering from diabetes and requiring amounts of delivered insulin of greater than 80 U/administration wherein the long acting insulin analogue is administered once daily as a single injection to said patient by injection in an amount of greater than 80 U/injection, wherein the long acting insulin analogue is degludec (IDeg) and wherein the composition is provided at a concentration of 200 U/mL or greater with respect to said long acting insulin analogue.

The invention will now further be described in by the following numbered paragraphs:
1. A long acting insulin analogue for use in reducing the risk of hypoglycaemia in a patient suffering from diabetes and requiring amounts of delivered insulin of greater than 80 U/administration, wherein the long acting insulin analogue is administered once daily as a single injection to said patient in an amount of greater than 80 U/administration and in the form of a pharmaceutical composition comprising said insulin at a concentration of 200 U/mL or greater,
   and wherein the long acting insulin analogue is a naturally occurring insulin or an insulin analogue having a side chain attached either to the α-amino group of the N-terminal amino acid residue of the B chain or to the ε-amino group of a Lys residue present in the B chain of the parent insulin, the side chain being of the general formula (I):

   -W-X-Y-Z

   wherein W is:
   - an α-amino acid residue having a carboxylic acid group in the side chain which residue forms, with one of its carboxylic acid groups, an amide group together with the α-amino group of the N-terminal amino acid residue of the B chain or together with the ε-amino group of a Lys residue present in the B chain of the parent insulin;
   - a chain composed of two, three or four α-amino acid residues linked together via amide bonds, which chain - via an amide bond - is linked to the α-amino group of the N-terminal amino acid residue of the B chain or to the ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain; or
   - a covalent bond from X to the α-amino group of the N-terminal amino acid residue of the B chain or to the ε-amino group of a Lys residue present in the B chain of the parent insulin;
   wherein X is:
   - -CO-;
   - -COCH(COOH)CO-;
   - -CON(CH₂COOH)CH₂CO-;
   - -CON(CH₂COOH)CH₂CON(CH₂COOH)CH₂CO-;
   - -CON(CH₂CH₂COOH)CH₂CH₂CO-;
   - -CON(CH₂CH₂COOH)CH₂CH₂CON(CH₂CH₂COOH)CH₂CH₂CO-;
   - -CONHCH(COOH)(CH₂)₄NHCO-;
   - -CON(CH₂CH₂COOH)CH₂CO-; or
   - -CON(CH₂COOH)CH₂CH₂CO-.
   that
   a) when W is an amino acid residue or a chain of amino acid residues, *via* a bond from the underscored carbonyl carbon forms an amide bond with an amino group in W, or
   b) when W is a covalent bond, *via* a bond from the underscored carbonyl carbon forms an amide bond with the N-terminal α-amino group in the B chain or with the ε-amino group of a Lys residue present in the B chain of the parent insulin;
   wherein Y is:
   • -(CH₂)ₘ- where m is an integer in the range of 6 to 32;
   • - a divalent hydrocarbon chain comprising 1, 2 or 3 -CH=CH- groups and a number of -CH₂- groups sufficient to give a total number of carbon atoms in the chain in the range of 10 to 32;
   • - a divalent hydrocarbon chain of the formula -(CH₂)ᵥC₆H₄(CH₂)_{W} - wherein v and w are integers or one of them is zero so that the sum of v and w is in the range of 6 to 30; and
   wherein Z is:
   - -COOH;
   - -CO-Asp;
   - -CO-Glu;
   - -CO-Gly;
   - -CO-Sar;
   - -CH(COOH)₂;
   - -N(CH₂COOH)₂;
   - -SO₃H; or
   - -PO₃H;
   and any Zn²⁺ complexes thereof, provided that when W is a covalent bond and X is -CO-, then Z is different from -COOH.
2. The long acting insulin analogue or pharmaceutical composition for use according to paragraph 1 wherein the side chain -W-X-Y-Z is attached to the ε-amino group of a Lys residue present in the B chain of the parent insulin.
3. The long acting insulin analogue or pharmaceutical composition for use according to any one of paragraphs 1 and 2 wherein the side chain -W-X-Y-Z is attached to the ε-amino group of a Lys residue present in position 29 of the B chain.
4. The long acting insulin analogue or pharmaceutical composition for use according to any one of paragraphs 1 and 3 wherein W is selected from the group consisting of α-Asp, β-Asp, α-Glu, γ-Glu, α-hGlu and δ-hGlu; preferably γ-Glu.
5. The long acting insulin analogue or pharmaceutical composition for use according to any one of paragraphs 1 and 4 wherein X is -CO-.
6. The long acting insulin analogue or pharmaceutical composition for use according to any one of paragraphs 1 and 5 wherein Y is a group of the formula - (CH₂)ₘ- where m is an integer in the range of from 6 to 32, from 8 to 20, from 12 to 20, or from 12-16.
7. The long acting insulin analogue or pharmaceutical composition for use according to any one of paragraphs 1 and 6 wherein Z is -COOH.
8. The long acting insulin analogue or pharmaceutical composition for use according to any one of paragraphs 1 and 7 wherein the amino acid residue at position B30 has been deleted from the insulin analogue.
9. The long acting insulin analogue or pharmaceutical composition for use according to any one of paragraphs 1 and 8 wherein the insulin analogue is des(30) human insulin.
10. The long acting insulin analogue or pharmaceutical composition for use according to any preceding paragraph wherein the long acting insulin analogue is selected from the list consisting of:
   N^{εB29}-(N^{α}-(HOOC(CH₂)₁₄CO)-γ-Glu) des(B30) human insulin (insulin degludec);
   N^{εB29}-(N^{α}-(HOOC(CH₂)₁₅CO)-γ-Glu) des(B30) human insulin;
   N^{εB29}-(N^{α}-(HOOC(CH₂)₁₆CO)-γ-Glu) des(B30) human insulin;
   N^{εB29}-(N^{α}-(HOOC(CH₂)₁₇CO)-γ-Glu) des(B30) human insulin;
   N^{εB29}-(N^{α}-(HOOC(CH₂)₁₈CO)-γ-Glu) des(B30) human insulin;
   N^{εB29}-(N^{α}-(HOOC(CH₂)₁₆CO)-γ-Glu-N-(γ-Glu)) des(B30) human insulin;
   N^{εB29}-(N^{α}-(Asp-OC(CH₂)₁₆CO)-γ-Glu) des(B30) human insulin;
   N^{εB29}-(N^{α}-(Glu-OC(CH₂)₁₄CO)-γ-Glu) des(B30) human insulin;
   N^{εB29}-(N^{α}-(Glu-OC(CH₂)₁₄CO-) des(B30) human insulin;
   N^{εB29}-(N^{α}-(Asp-OC(CH₂)₁₆CO-) des(B30) human insulin;
   N^{εB29}-(N^{α}-(HOOC(CH₂)₁₆CO)-α-Glu-N-(β-Asp)) des(B30) human insulin;
   N^{εB29}-(N^{α}-(Gly-OC(CH₂)₁₃CO)-γ-Glu) des(B30) human insulin;
   N^{εB29}-(N^{α}-(Sar-OC(CH₂)₁₃CO)-γ-Glu) des(B30) human insulin;
   N^{εB29}-(N^{α}-(HOOC(CH₂)₁₃CO)-γ-Glu) des(B30) human insulin;
   (N^{εB29}-(N^{α}-(HOOC(CH₂)₁₃CO)-β-Asp) des(B30) human insulin;
   N^{εB29}-(N^{α}-(HOOC(CH₂)₁₃CO)-α-Glu) des(B30) human insulin;
   N^{εB29}-(N^{α}-(HOOC(CH₂)₁₆CO)-γ-D-Glu) des(B30) human insulin;
   N^{εB29}-(N^{α}-(HOOC(CH₂)₁₄CO)-β-D-Asp) des(B30) human insulin;
   N^{εB29}-(N-HOOC(CH₂)₁₆CO-β-D-Asp) des(B30) human insulin;
   N^{εB29}-(N-HOOC(CH₂)₁₄CO-IDA) des(B30) human insulin;
   N^{εB29}-[N-(HOOC(CH₂)₁₆CO)-N-(carboxyethyl)-Gly] des(B30) human insulin;
   N^{εB29}-[N-(HOOC(CH₂)₁₄CO)-N-(carboxyethyl)-Gly] des(B30) human insulin; and
   N^{εB29}-[N-(HOOC(CH₂)₁₄CO)-N-(carboxymethyl)-β-Ala] des(B30) human insulin.
11. The long acting insulin analogue or pharmaceutical composition for use according to any preceding paragraph wherein the long acting insulin analogue is insulin degludec.
12. The long acting insulin analogue or pharmaceutical composition for use according to any preceding paragraph wherein the long acting insulin analogue is administered in an amount from about 80 U/administration to about 160 U/administration.
13. The long acting insulin analogue or pharmaceutical composition for use according to any preceding paragraph wherein the long acting insulin analogue is administered by injection, preferably subcutaneous injection.
14. The long acting insulin analogue or pharmaceutical composition for use according to any preceding paragraph wherein the long acting insulin analogue is administered via a pen injection device.
15. The long acting insulin analogue or pharmaceutical composition for use according to any preceding paragraph wherein the patient is suffering from type-2 diabetes.
16. The long acting insulin analogue or pharmaceutical composition for use according to any preceding paragraph wherein the patient is insulin naive.
17. The long acting insulin analogue or pharmaceutical composition for use according to any preceding paragraph wherein the patient has previously been treated with OADs.
18. The long acting insulin analogue or pharmaceutical composition for use according to any preceding paragraph wherein the patient is undergoing concomitant therapy with oral antidiabetic drugs, preferably selected from metformin, dipeptidyl peptidase-IV inhibitors and combinations thereof.
19. The long acting insulin analogue or pharmaceutical composition for use according to any preceding paragraph wherein the patient requires greater than 80 U/administration of delivered insulin.
20. The long acting insulin analogue or pharmaceutical composition for use according to any preceding paragraph wherein the patient requires greater than 80 U/day of delivered insulin.
21. The long acting insulin analogue or pharmaceutical composition for use according to any preceding paragraph wherein the patient requires between 80 and 160 U/day of delivered insulin.
22. The long acting insulin analogue or pharmaceutical composition for use according to any preceding paragraph wherein the patient has a body/mass index (BMI) of 30kg/m² or more.
23. The long acting insulin analogue or pharmaceutical composition for use according to any preceding paragraph wherein the patient has a body/mass index (BMI) of between 30 and 45 kg/m².
24. The long acting insulin analogue or pharmaceutical composition for use according to any preceding paragraph wherein a beneficial glycaemic effect is determined by a 0.5% reduction in HbA_{1c} compared to baseline HbA_{1c}; preferably 0.7% reduction, more preferably 0.9% reduction, more preferably 1.0% reduction, more preferably 1.2% reduction, more preferably 1.3% reduction.
25. The long acting insulin analogue or pharmaceutical composition for use according to any preceding paragraph wherein a beneficial glycaemic effect is determined by a decrease of the level of HbA1c to about 7% or less, preferably to about 6.5% or less.
26. The long acting insulin analogue or pharmaceutical composition for use according to any preceding paragraph for use wherein the hypoglycaemia is nocturnal hypoglycaemia.
27. The long acting insulin analogue or pharmaceutical composition for use according to any preceding paragraph wherein the risk of hypoglycaemia is reduced to a rate of hypoglycaemic episodes of less than 2 episodes/exposure in a year; preferably less than 1.5 episodes/exposure in a year; more preferably less than 1 episodes/exposure year; more preferably less than 0.5 episodes/exposure in a year.
28. The long acting insulin analogue or pharmaceutical composition for use according to any preceding paragraph wherein the risk of hypoglycaemia is reduced to a rate of hypoglycaemic episodes of less than 1.0 episodes/exposure in a year; preferably less than 0.7 episodes/exposure in a year; preferably less than 0.5 episodes/exposure in a year; preferably less than 0.3 episodes/exposure in a year; preferably less than 0.1 episodes/exposure in a year.
29. The long acting insulin analogue for the use according to any preceding paragraph wherein said long acting insulin analogue is in the form of a pharmaceutical composition wherein said pharmaceutical composition also comprises at least one pharmaceutically acceptable excipient, diluent or carrier.
30. The pharmaceutical composition according to any preceding paragraph wherein the long acting insulin analogue is provided at a concentration of about 200 U/mL.
31. The long acting insulin analogue for the use according to any preceding paragraph wherein the long acting insulin analogue is provided in a pharmaceutical composition at a concentration of about 200 U/mL.
32. The long acting insulin analogue for the use according to any preceding paragraph wherein the long acting insulin analogue is insulin degludec and wherein the insulin degludec is provided in a pharmaceutical composition at a concentration of about 200 U/mL.
33. The long acting insulin analogue or the pharmaceutical composition according to any preceding paragraph for use in a patient requiring amounts of delivered insulin of greater than 80 U/administration.
34. The long acting insulin analogue or the pharmaceutical composition according to any preceding paragraph for use in a patient requiring amounts of delivered insulin of greater than 80 U/administration and wherein the administration is a once daily administration.
35. The long acting insulin analogue or the pharmaceutical composition according to any preceding paragraph for use in a patient requiring amounts of delivered insulin of greater than 80 U/administration, wherein the administration is by injection and wherein the long acting insulin analogue is administered to said patient in an amount of greater than 80 U/injection.
36. The long acting insulin analogue or the pharmaceutical composition according to any of paragraphs 1 to 11, and paragraph 31 and any one of paragraphs 33 to 35 wherein the long acting insulin analogue has an insulin action of up to at least 24h, i.e. of at least 24h.
37. The long acting insulin analogue or the pharmaceutical composition according to any of paragraphs 1 to 11, and paragraph 31 and any one of paragraphs 33 to 35 wherein the long acting insulin analogue has an insulin action of up to at least 30h or 36h, i.e. of at least 30h or 36h.
38. The long acting insulin analogue or the pharmaceutical composition according to any of paragraphs 1 to 11, and paragraph 31 and any one of paragraphs 30 to 35 wherein the long acting insulin analogue has an insulin action of up to at least 42h, i.e. of at least 42h.
39. The long acting insulin analogue or the pharmaceutical composition according to any of paragraphs 33 to 35 wherein the long acting insulin analogue is as defined in paragraph 1.
40. The long acting insulin analogue or the pharmaceutical composition according to paragraph 1, paragraph 31 and any of paragraphs 33 to 35.
41. The long acting insulin analogue or the pharmaceutical composition for use according to paragraph 1 in combination with any of paragraphs 32 to 35.
42. The pharmaceutical composition according to paragraph 32 in combination with any of paragraphs 33 to 35.
43. The long acting insulin analogue for the use according to any preceding claim wherein said long acting insulin analogue is in the form of a pharmaceutical composition wherein said pharmaceutical composition comprises said long acting insulin analogue and at least one pharmaceutically acceptable excipient, diluent or carrier.
44. Use of a long acting insulin analogue according to any of the preceding paragraphs in the preparation of a medicament for the treatment of a patient suffering from diabetes and requiring amounts of delivered insulin of greater than 80 U of delivered insulin per administration, or for use in reducing the risk of hypoglycaemia or for use in providing a beneficial glycaemic control to such patient.
45. Method of treatment of a patient suffering from diabetes and requiring high amounts of delivered insulin, or method of reducing the risk of hypoglycaemia or of providing a beneficial glycaemic control to a patient suffering from diabetes and requiring amounts of delivered insulin of greater than 80 U of delivered insulin per administration, comprising the administration of a long acting insulin analogue according to any of the preceding paragraphs.
46. A method of reducing the risk of hypoglycaemia in a patient suffering from diabetes and requiring high amounts of delivered insulin which comprises administering to said patient by injection a long acting insulin analogue and in an amount of greater than 80 U/ injection, wherein the long acting insulin analogue is insulin degludec (IDeg).
47. Use of a long acting insulin analogue in the preparation of a medicament for reducing the risk of hypoglycaemia in a patient suffering from diabetes and requiring high amounts of delivered insulin, wherein the long acting insulin analogue is administered to said patient by injection and in an amount of greater than 80 U/ injection, wherein the long acting insulin analogue is insulin degludec (IDeg).
48. A long acting insulin analogue or a method according to any one of previous paragraphs wherein insulin action is keeping blood sugar levels at a steady and stable level, for up to any one of at least 24 hours, at least 30 hours, at least 36 hours, at least 42 hours, at least 48 hours.
49. The long acting insulin analogue for the use according to any of previous paragraphs and providing beneficial glycaemic control to said patient.
50. A long acting insulin analogue for use according to paragraph 1 in treating said patient and reducing the risk of hypoglycaemia of said patient.
51. A long acting insulin analogue for use according to paragraph 1 in providing beneficial glycaemic control and reducing the risk of hypoglycaemia of said patient.

The present invention is further illustrated by the following examples.

### Examples

### Synthesis of N^{εB29}-(N^{α}-(HOOC(CH₂)₁₄CO)-γ-Glu) des(B30) human insulin

Example 1 of WO 2005/012347 is referenced.

### Clinical Trial 1

### Trial design

This phase 3a, 26-week, randomized, controlled, open-label, multinational, treat-to-target, non-inferiority trial compared the efficacy and safety of IDeg U-200 (i.e. 200 U/mL) and IGlar (100 U/mL) both administered OD in combination with metformin (met) ± DPP-4 inhibitor in insulin-naive participants with T2DM previously treated with oral antidiabetic drugs OAD(s), who qualified for intensification of treatment. The trial was open-label because the pen devices used to administer the basal insulins were distinctively different and blinding was, therefore, impossible.

The study was completed in compliance with the Declaration of Helsinki and the International Conference on Harmonisation (ICH) Good Clinical Practice Guidelines; institutional review boards reviewed and approved the protocol for each study site; and all patients provided written informed consent before participation in the trial.^{5,6} The trial is registered at ClinicalTrials.gov as NCT01068665.

### Participants

Participants who were insulin-naive adults with T2DM for at least 6 months, HbA_{1c} 7-10% (inclusive), BMI ≤ 45 kg/m², and previously treated with metformin with or without additional OADs for at least 3 months were eligible to participate in the study. Key exclusion criteria included thiazolidinedione (TZD), exenatide or liraglutide use within 3 months of participation in the trial, cardiovascular disease (e.g., stroke, myocardial infarction, unstable angina pectoris) within 6 months of the trial, uncontrolled hypertension (systolic blood pressure [BP] ≥180 mm Hg and/or diastolic BP ≥100 mm Hg), impaired liver function (alanine aminotransferase [ALAT] ≥2.5 times the upper limit of normal), impaired renal function (serum-creatinine ≥125 µmol/L or ≥1.4 mg/dL for males and ≥110 µmol/L or ≥1.3 mg/dL for females), recurrent severe hypoglycaemia (greater than one episode requiring assistance in the previous 12 months) or hypoglycaemic unawareness, hospitalization for diabetic ketoacidosis within 6 months of the trial, and proliferative retinopathy or maculopathy.

### Treatments

Using the interactive voice/web response system, eligible participants were randomized 1:1 to either once-daily IDeg U-200 (IDeg 200 U/mL, 3 mL FlexTouch®, Novo Nordisk, Bagsværd, Denmark) or once-daily IGlar (Lantus® 100 U/mL, 3 mL SoloStar® Sanofi Aventis U.S. LLC) and continued metformin ± DPP-4 inhibitor treatment. Trial participants were instructed to continue the same total daily dose of metformin and DPP-4 inhibitor treatment as before the start of the trial.

Insulin degludec was administered once-daily with the main evening meal and, consistent with its product labelling, IGlar was administered once-daily (OD) at the same time each day. Either treatment was injected subcutaneously in the thigh, upper arm or abdomen. The starting dose for each was 10 U, and during the treatment period, the insulin dose was systematically titrated using a treat-to-target approach striving for a pre-breakfast self-measured plasma glucose (SMPG) level of 4.0-4.9 mmol/L (71-89 mg/dL). Both IDeg and IGlar were similarly titrated once weekly in 2 U increments. The treatment period was 26 weeks and doses were individually titrated in an effort to achieve the same glucose targets with both treatments.

### Primary and secondary endpoints

The primary endpoint for this study was change in HbA_{1c} (%) from baseline after 26 weeks of treatment. Secondary confirmatory endpoints tested were number of treatment-emergent confirmed hypoglycaemic episodes, change from baseline in central laboratory-measured fasting plasma glucose (FPG), within subject variability as measured by coefficient of variation (%), and frequency of participants achieving HbA_{1c} <7% without hypoglycaemic episodes. The supportive secondary endpoints included 9-point SMPG profiles, frequency of participants achieving HbA_{1c} <7%, and Health-Related Quality of Life (HRQoL; Short Form 36 [SF-36 v.2] questionnaire) scores. The safety assessments were adverse events, hypoglycaemic episodes, insulin dose, body weight, physical examination, vital signs, fundoscopy, electrocardiogram (ECG) and laboratory tests (including antibodies). Confirmed hypoglycaemic episodes were defined as episodes with a PG value of <3.1 mmol/L (56 mg/dL) and severe episodes requiring assistance.⁷ Hypoglycaemic episodes occurring between 00:01 h and 05:59 h (inclusive) were classified as nocturnal.

### Statistical analysis

Analyses of all efficacy endpoints were based on the full analysis set (FAS) which included all randomized participants. The safety analysis set included all participants who received at least one dose of the investigational product or the comparator. Missing values were imputed using the last observation carried forward (LOCF) method.

The primary objective of this trial was to confirm the non-inferiority of IDeg U-200 OD to IGlar OD as assessed by change in HbA_{1c} from baseline after 26 weeks of treatment. Non-inferiority was confirmed if the upper limit of the 95% confidence interval (CI) for the treatment difference was ≤0.4%.

Treatment difference in change from baseline in HbA_{1c} after 26 weeks was analyzed using an Analysis of Variance (ANOVA) model with treatment, antidiabetic treatment at screening, sex and region (Europe, North America, or South Africa) as fixed factors, and age and baseline HbA_{1c} as covariates. Responder (HbA_{1c}) analysis was based on a logistic regression model using treatment, antidiabetic therapy at screening, sex and region as fixed factors, and age and baseline HbA_{1c} as covariates. The number of treatment-emergent confirmed hypoglycaemic episodes was analyzed using a negative binomial regression model including treatment, antidiabetic therapy at screening, sex and region as fixed factors, and age as covariate. A similar model was used for analysis of treatment-emergent confirmed hypoglycaemic episodes in participants requiring ≥80 U of insulin by the end of trial. However, nocturnal confirmed hypoglycaemic episodes for participants requiring ≥80 U by the end of trial was analyzed using a Poisson model with only treatment as a fixed factor.

### Results

### Participant characteristics

A total of 697 people were screened for this study, of which 237 were screening criteria failures, and the remaining 460 participants were randomly assigned (1:1) to the IDeg U-200 and IGlar treatment groups. Three of 460 participants were randomized in error and were withdrawn from the trial without any trial treatments. Accordingly, 457 (IDeg: 228 and IGlar: 229) and 456 (IDeg: 228 and IGlar: 228) participants were exposed to treatment and comprised the intent-to-treat and safety populations. One randomized subject in the IGlar group withdrew consent before the trial drug was given. Both treatment groups had similar baseline characteristics and demographics **(Table 1).**

**Table 1. Demographics and baseline characteristics**

| **Characteristic** | **IDeg U-200 OD** | **IGlar OD** |
|---|---|---|
| Participants in the full analysis set, N | 228 | 229 |
| Participants in the safety analysis set, N | 228 | 228* |
| Female, n (%) | 109 (47.8%) | 105 (45.9%) |
| *Race*, n (%) | | |
| White | 180 (78.9%) | 178 (77.7%) |
| Black | 31 (13.6%) | 32 (14.0%) |
| Asian (Indian or non-Indian) | 8 (3.5%) | 9 (3.9%) |
| Other | 24 (10.5%) | 25 (10.8%) |
| Ethnicity: Hispanic or Latin American, n (%) | 20 (8.8%) | 16 (7.0%) |
| Age, years | 57.8 (±9.0) | 57.3 (±9.4) |
| Body weight, kg | 92.2 (±18.5) | 92.7 (±18.4) |
| BMI, kg/m² | 32.2 (±5.4) | 32.7 (±5.3) |
| Duration of diabetes, years | 8.4 (±6.7) | 8.0 (±5.6) |
| HbA_{1c}, % | 8.3 (±1.0) | 8.2 (±0.9) |
| FPG, mg/dL | 172.8 (±52.2) | 174.6 (±46.8) |
| Systolic blood pressure, mmHg | 131.2 (±13.9) | 131.0 (±13.6) |
| Diastolic blood pressure, mmHg | 78.1 (±8.3) | 79.2 (±8.4) |
| HDL cholesterol, mg/dL | 43.3 (±11.6) | 42.9 (±11.2) |
| LDL cholesterol, mg/dL | 92.8 (±38.3) | 94.4 (±39.8) |
| Total cholesterol, mg/dL | 170.5 (±43.7) | 172.9 (±52.6) |
| Triglycerides, mg/dL | 184.07 (±215.93) | 184.96 (±221.24) |
| *OADs at screening* | | |
| Metformin | 228 (100.0%) | 229 (100·0%) |
| SU | 149 (65.3%) | 151 (65.9%) |
| DPP-4 inhibitor^{†} | 39 (17.1%) | 34 (14.8%) |
| Glinide | 0 (0.0%) | 4 (1.7%) |
| Alpha-glucosidase inhibitor | 4 (1.8%) | 1 (0.4%) |
| *Antidiabetic treatment at screening* | | |
| 1 OAD | 62 (27.2%) | 70 (30.6%) |
| 2 OADs | 141 (61.8%) | 133 (58.1%) |
| >2 OADs | 25 (11.0%) | 26 (11.4%) |

| | | |
|---|---|---|
| *One randomized participants withdrew consent and was never administered any drug product. ^{†}In countries where DPP-4 inhibitor treatment did not have an indication of combination with insulin treatment, 17 participants in each treatment arm discontinued their DPP-4 inhibitor treatment at randomization. Data are presented as number (%) or mean (SD). | | |

### Efficacy

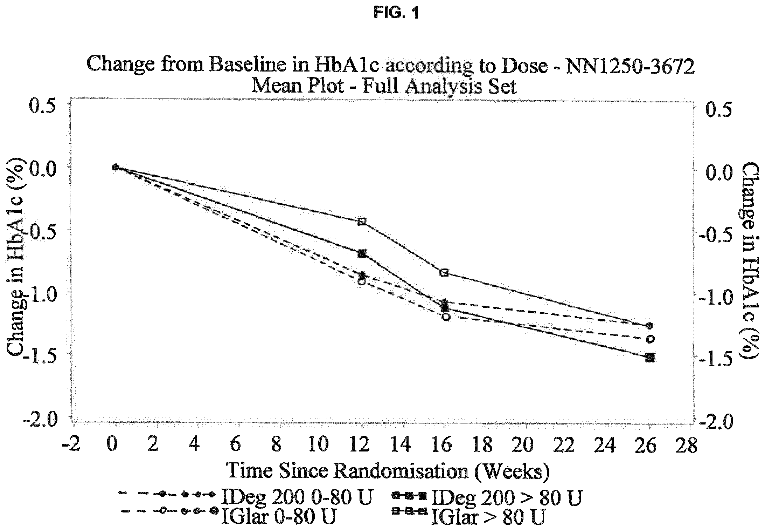

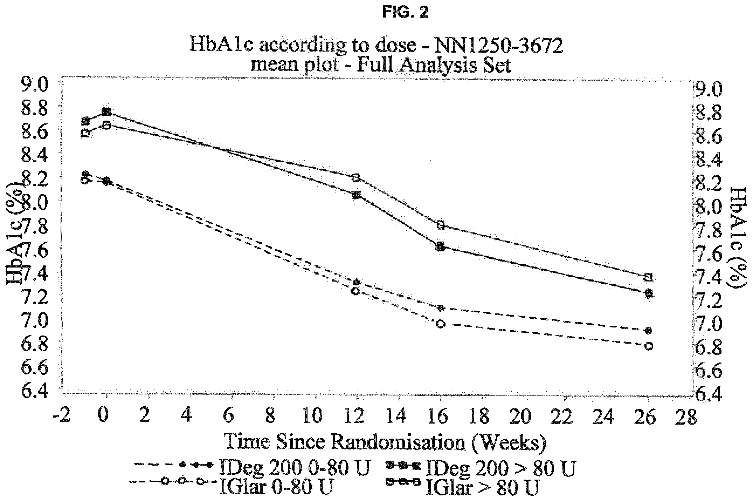

Considering the whole population of patients in the trial, glycaemic control, in terms of change in HbA_{1c} from baseline, improved with both IDeg U-200 and IGlar after 26 weeks of treatment. Mean HbA_{1c} decreased by 1.3% ± 1.01 (mean ± SD) for both treatment groups with an estimated treatment difference (ETD) IDeg-IGlar: 0.04 [95% CI: -0.11; 0.19]), which confirmed non-inferiority of IDeg to IGlar.
Looking into subgroups of patients receiving either up to 80 units or above 80 units of delivered insulin, HbA_{1c} reductions for subjects receiving more than 80 units were similar or tended to be greater for subjects treated with IDeg U-200 U/mL than comparator IGlar as shown in **Table 2** and **Table 3** below and corresponding graphs in **Figure 1** and **Figure 2****.**

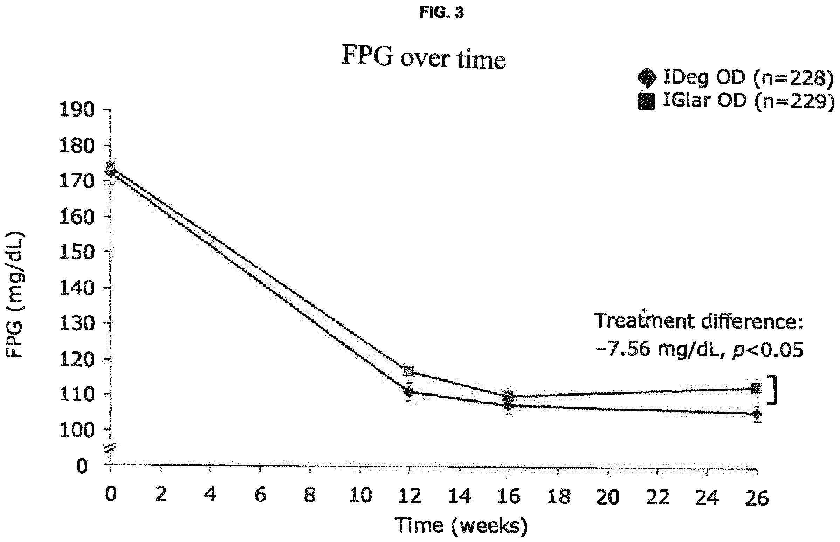

In the whole population of patients, insulin degludec resulted in a statistically significantly greater FPG reduction than IGlar after 26 weeks of treatment **(****Figure 3****).** Central laboratory-measured FPG decreased by 4.0 mmol/L to 5.7 mmol/L with IDeg, and by 3.6 mmol/L to 6.0 mmol/L with IGlar (ETD: -0.42 [95% CI: -0.78; -0.06]).

**Table 2. Change from Baseline in HbA_{1c} according to dose - Full Analysis Set**

| | | IDeg U-200 OD | | IGlar OD | |
|---|---|---|---|---|---|
| | Week | N | Mean | N | Mean |
| End of trial dose 0-80 U | 0 | 179 | 0.00 | 178 | 0.00 |
| | 12 | 179 | -0.85 | 178 | -0.91 |
| | 16 | 179 | -1.07 | 178 | -1.18 |
| | 26 | 179 | -1.25 | 178 | -1.36 |
| End of trial dose > 80 U | 0 | 48 | 0.00 | 47 | 0.00 |
| | 12 | 48 | -0.68 | 47 | -0.43 |
| | 16 | 48 | -1.12 | 47 | -0.83 |
| | 26 | 48 | -1.51 | 47 | -1.26 |

| | | | | | |
|---|---|---|---|---|---|
| N: Number of subjects in FAS | | | | | |

**Table 3. HbA_{1c} according to dose - summary - Full Analysis Set**

| | | IDeg U-200 OD | | IGlar OD | |
|---|---|---|---|---|---|
| | Week | N | Mean | N | Mean |
| End of trial dose 0-80 U | -1 | 179 | 8.22 | 178 | 8.17 |
| | 0 | 179 | 8.17 | 178 | 8.15 |
| | 12 | 179 | 7.32 | 178 | 7.25 |
| | 16 | 179 | 7.10 | 178 | 6.97 |
| | 26 | 179 | 6.92 | 178 | 6.79 |
| End of trial dose > 80 U | -1 | 48 | 8.67 | 47 | 8.57 |
| | 0 | 48 | 8.75 | 47 | 8.64 |
| | 12 | 48 | 8.06 | 47 | 8.21 |
| | 16 | 48 | 7.63 | 47 | 7.81 |
| | 26 | 48 | 7.24 | 47 | 7.38 |

| | | | | | |
|---|---|---|---|---|---|
| N: Number of subjects in FAS | | | | | |

### Dosing

After 26 weeks of treatment, the mean daily insulin doses were similar between the IDeg U-200 and IGlar groups (59 U [0.62 U/kg] and 63 U [0.66 U/kg], respectively); mean ratio IDeg/IGlar: 0.95 [0.94]). For both treatment groups, the largest increase in insulin dose was observed during the first few weeks of the trial, but continued to increase gradually throughout the trial. The percentage of participants who required more than 80 U of insulin was 21.2% and 20.9%, and more than 160 U of insulin daily was 0.9% and 0.9% in the IDeg and IGlar groups at the end of trial, respectively. The titration algorithm was closely adhered to as indicated by the close to 0 U mean and median differences between the titration algorithm dose and the prescribed dose.

### Safety

No participants in either of the treatment groups reported episodes of severe hypoglycaemia.

The overall percentage of participants who experienced one or more confirmed hypoglycaemic episodes during the treatment period was 28.5% with IDeg U-200 and 30.7% with IGlar **(Table 4).** The event rates of confirmed hypoglycaemia overall with IDeg U-200 and IGlar were 1.22 and 1.42 episodes/patient-year, respectively; (estimated rate ratio (ERR) IDeg/IGlar: 0.86 [95% CI: 0.58; 1.28], p=0.46).

Analysed by dosing group, the results show that the percentage of participants who experienced one or more confirmed hypoglycaemic episodes during the treatment period was lower in those patients receiving >80 U daily of a long acting insulin analogue at the end of trial than those patients which required up to 80 U daily. Furthermore, the rate of confirmed hypoglycaemic episodes was much lower in the groups receiving >80 U daily of a long acting insulin analogue at the end of trial than those patients which were administered up to 80 U daily at the end of trial. (Table 4)

In particular, when IDeg U-200 was administered, in those patients receiving >80 U daily at the end of the trial, confirmed hypoglycaemic episodes were experienced in 14.6% of patients compared to 32.4% of those patients receiving 0-80 U daily . Furthermore, the rate of episodes per exposure year was 0.38 in the >80 U patient group, lower than that of the 0-80U patient group which showed a rate of 1.46 episodes per exposure year. (Table 4)

Similarly, when IGlar U-100 was administered, in those patients receiving >80 U daily at the end of the trial, confirmed hypoglycaemic episodes were experienced in 17.0% of patients compared to 34.8% of those patients receiving 0-80 U daily . Furthermore, the rate of episodes per exposure year was 0.74 in the >80 U patient group, lower than that of the 0-80U patient group which showed a rate of 1.61 episodes per exposure year. (Table 4)

A total of 6.1% and 8.8% of participants in the IDeg and IGlar treatment groups, respectively, experienced nocturnal confirmed hypoglycaemic episodes with rates of 0.18 and 0.28 episodes/patient-year, respectively; (ERR IDeg/IGlar: 0.64 [95% CI: 0.30; 1.37], p=0.25).

Analysed by dosing group, the results show that the percentage of participants who experienced one or more nocturnal confirmed hypoglycaemic episodes during the treatment period was lower in the those patients receiving >80 U daily of a long acting insulin analogue by trial end than those patients receiving up to 80 U daily. The rate of nocturnal confirmed hypoglycaemic episodes was much lower in the groups requiring >80 U daily of a long acting insulin analogue than those patients which were administered up to 80 U daily. (Table 4)

In particular, when IDeg U-200 was administered, in those patients receiving >80 U daily at the end of the trial, nocturnal confirmed hypoglycaemic episodes were experienced in 2.1% of patients compared to 7.3% of those patients receiving 0-80 U daily . Furthermore, the rate of episodes per exposure year was 0.04 in the >80 U patient group, lower than that of the 0-80 U patient group which showed a rate of 0.22 episodes per exposure year. (Table 4)

Similarly, when IGlar U-100 was administered, in those patients receiving >80 IU daily at the end of the trial, confirmed hypoglycaemic episodes were experienced in 2.1% of patients compared to 10.7% of those patients receiving 0-80 U daily. Furthermore, the rate of episodes per exposure year was 0.13 in the >80 U patient group, lower than that of the 0-80 U patient group which showed a rate of 0.32 episodes per exposure year. (Table 4)

**Table 4. Observed episodes of Hypoglycaemia per year of exposure**

| **Confirmed Hypoglycaemic Episodes** | **Insulin analogue** | **SAS** | **N** | **%** | **E** | **R** |
|---|---|---|---|---|---|---|
| All | IDeg 200 U/mL | 228 | 65 | 28.51 | 129 | 1.22 |
| | IGlar 100 U/mL | 228 | 70 | 30.70 | 152 | 1.42 |
| | Total | 456 | 135 | 29.61 | 281 | 1.32 |
| End of trial dose 0-80U | IDeg 200 U/mL | 179 | 58 | 32.40 | 120 | 1.46 |
| | IGlar 100 U/mL | 178 | 62 | 34.83 | 135 | 1.61 |
| | Total | 357 | 120 | 33.61 | 255 | 1.53 |
| End of trial dose >80U | IDeg 200 U/mL | 48 | 7 | 14.58 | 9 | 0.38 |
| | IGlar 100 U/mL | 47 | 8 | 17.02 | 17 | 0.74 |
| | Total | 95 | 15 | 15.79 | 26 | 0.56 |

| **Nocturnal Confirmed Hypoglycaemic Episodes** | | | | | | |
|---|---|---|---|---|---|---|
| All | IDeg 200 U/mL | 228 | 14 | 6.14 | 19 | 0.18 |
| | IGlar 100 U/mL | 228 | 20 | 8.77 | 30 | 0.28 |
| | Total | 456 | 34 | 7.46 | 49 | 0.23 |
| End of trial dose 0-80U | IDeg 200 U/mL | 179 | 13 | 7.26 | 18 | 0.22 |
| | IGlar 100 U/mL | 178 | 19 | 10.67 | 27 | 0.32 |
| | Total | 357 | 32 | 8.96 | 45 | 0.27 |

| **Confirmed Hypoglycaemic Episodes** | **Insulin analogue** | **SAS** | **N** | **%** | **E** | **R** |
|---|---|---|---|---|---|---|
| End of trial dose >80U | IDeg 200 U/mL | 48 | 1 | 2.08 | 1 | 0.04 |
| | IGlar 100 U/mL | 47 | 1 | 2.13 | 3 | 0.13 |
| | Total | 95 | 2 | 2.11 | 4 | 0.09 |

| | | | | | | |
|---|---|---|---|---|---|---|
| SAS: Number of subjects in safety analysis set N: number of subjects experiencing at least one episode %: Percentage of subjects experiencing at least one episode E: Number of episodes R: Rate of episodes per exposure year | | | | | | |

As shown in Table 5, the estimated rate of hypoglycaemic episodes per 100 years is lower in the patient group whom were receiving an end of trial dose of greater than 80 U. Furthermore, as can be seen from the estimate rate ratio, IDeg U-200 was more effective in reducing the risk of hypoglycaemia than IGlar, particularly nocturnal hypoglycaemia.

**Table 5. Estimated rates of hypoglycaemic episodes per 100 years of exposure**

| **End of Trial Dose** | **Subgroup of Hypoglycaemia** | **Estimated Rate of Episodes per 100 year** | | **Estimated Rate-Ratio IDeg 200U/ml / IGlar 100U/ml** | |
|---|---|---|---|---|---|
| | | **IDeg 200U/ml** | **IGlar 100U/ml** | **RR** | **95% CI** |
| 0-80U | Confirmed Hypoglycaemia | 134.7 | 148.6 | 0.907 | [0.602; 1.364] |
| | Nocturnal Confirmed Hypoglycaemia | 14.5 | 21.9 | 0.660 | [0.305; 1.430] |
| >80U | Confirmed Hypoglycaemia | 32.3 | 41.8 | 0.775 | [0.192; 3.128] |
| | Nocturnal Confirmed Hypoglycaemia | 4.1 | 12.6 | 0.324 | [0.034; 3.114] |

| | | | | | |
|---|---|---|---|---|---|
| The number of episodes is analysed using a negative binomial regression model using a log link and the logarithm of the exposure time as offset. The model includes treatment, sex, region and antidiabetic treatment at screening as fixed effects and age as covariate. For nocturnal hypos and dose >80U a Poisson model was used with treatment as fixed effect due to very sparse data. | | | | | |

### Discussion

Results from this 26-week, treat-to-target study demonstrate that IDeg U-200 improves glycaemic control, as measured by HbA_{1c}, and is non-inferior to IGlar in insulin-naive patients with T2DM requiring intensification of treatment beyond oral therapy. Due to the use of the low volume IDeg U-200, participants in the IDeg treatment group received their required insulin dose with smaller injection volumes and participants who required more than 80 U of basal insulin per day (approximately 20% in both groups) were able to administer the full dose in a single injection for those in the IDeg-treated group rather than two consecutive injections for IGlar-treated participants.

The similar HbA_{1c} levels achieved in this study, reflective of the treat-to-target study design, allows a comparison of potential differences in the safety profile of these two basal insulins. Both insulins demonstrated a total absence of severe hypoglycaemic episodes and a particularly low number of confirmed and nocturnal confirmed hypoglycaemic episodes when used in a structured, treat-to-target titration designed to reach a fasting glucose target of <90 mg/dL. Historically, hypoglycaemia has been a barrier to good glycaemic control in T1DM; however, it is often not emphasized in the T2DM population, possibly due to the lower risk of occurrence in this population compared with T1DM patients.⁸ Given the consequences that result from the occurrence of hypoglycaemic episodes, including, but not limited to, deterioration of the patients' health, the economic impact on patients and the healthcare system, and decreased patient adherence to treatment regimens, any reduction of hypoglycaemia would arguably have beneficial implications.

Furthermore, it has been surprisingly found that long acting insulin analogues show a reduced risk of hypoglycaemia, in patients to whom greater than 80 U daily was administered by the end of the trial. Treatment with IDeg U-200 tends to produce a greater reduction in the risk of hypoglycaemia in these patients than IGlar. In particular, IDeg U-200 was surprisingly found to lower the risk of both confirmed and nocturnal confirmed hypoglycaemia compared to IGlar at comparable or better glycaemic control in subjects needing high daily insulin doses of >80 U. Thus, IDeg U-200 is particularly beneficial for patients in need of high doses because compared to IGlar treatment, treatment with IDeg U-200 provides to these group of patients both the best improvement of glycaemic control (largest change from baseline and lowest end of trial HbA_{1c}) and the lowest risk of hypoglycaemic episodes.

### Clinical Trial 2

### Trial Design

This was a confirmatory 22-week, randomised (1:1), open labelled, stratified, multi-centre, two armed parallel group, treat-to-target, controlled trial comparing efficacy and safety of IDeg 200 U/mL with IDeg 100 U/mL, both in combination with pre-trial OAD treatment, in subjects with type 2 diabetes mellitus.

### Participants

Participants were eligible to participate in the study if they had Type 2 diabetes (diagnosed clinically for 24 weeks prior to randomisation); had level of HbA_{1c} 7-10% (inclusive), BMI ≤ 45 kg/m^{2;} and were currently being treated with basal-only insulin (no prandial insulin) consisting of either insulin detemir OD, insulin glargine OD or neutral protamine Hagedorn (NPH) insulin OD or twice daily (BID) for at least 12 weeks prior to randomisation, in combination with stable doses of OAD(s) (metformin, insulin secretagogue [sulfonylurea or glinide], alpha-glucosidase inhibitor, pioglitazone or DPP-IV inhibitor) in any approved (according to label) dose or combination. Stable OAD doses were defined as unchanged doses for at least 12 weeks prior to randomisation.

Key exclusion criteria included rosiglitazone or GLP-1 receptor agonist use within the 12 weeks prior to participation in the trial; cardiovascular disease (e.g., stroke, myocardial infarction, unstable angina pectoris) within 24 weeks of the trial, uncontrolled hypertension (systolic blood pressure [BP] ≥180 mm Hg and/or diastolic BP ≥100 mm Hg), impaired liver function (alanine aminotransferase [ALAT] ≥2.5 times the upper limit of normal), impaired renal function (serum-creatinine ≥125 µmol/L or ≥1.4 mg/dL for males and ≥110 µmol/L or ≥1.3 mg/dL for females), recurrent severe hypoglycaemia (greater than one episode requiring assistance in the previous 12 months) or hypoglycaemic unawareness, and proliferative retinopathy or maculopathy requiring treatment.

### Treatments

Subjects found eligible for the trial were to be randomised in a 1:1 manner into one of the two treatment arms:
- IDeg 200 U/mL OD in combination with unchanged pre-trial OAD treatment
- IDeg 100 U/mL OD in combination with unchanged pre-trial OAD treatment

The total treatment period with trial products was to be 22 weeks. IDeg 200 U/mL and IDeg 100 U/mL were provided by Novo Nordisk A/S. During the screening and trial treatment period it was not allowed to start any other antidiabetic treatment, change the pre-trial OAD doses or start treatment with any medication known to interfere significantly with glucose metabolism, unless for safety reasons at the investigator's opinion.

IDeg 100 U/mL and IDeg 200 U/mL was to be injected subcutaneously either in the thigh, upper arm (deltoid region) or abdomen OD at any time of the day as preferred by the subject. The injection time was to remain the same throughout the trial treatment period.

During the trial treatment period the insulin dose was to be titrated once weekly by the investigator based upon the subject's SMPG levels and the Insulin Titration Guideline. The insulin dose adjustments were to aim at a pre-breakfast SMPG value between 4.0 and 5.0 mmol/L (71 and 90 mg/dL).

The starting dose of trial insulin was to be based on a 1:1 transfer of the total daily pre-randomisation insulin therapy dose. No maximum insulin dose was specified.

All subjects were to continue OAD treatment at pre-trial dose level and dosing frequency. The dose and dosing frequency of OAD(s) was not be changed at any time during the screening and trial treatment period, unless for safety reasons.

### Primary and Secondary Endpoints

The primary objective of this trial was to confirm the efficacy of IDeg 200 U/mL in controlling glycaemia by comparing the difference in change from baseline in HbA1c between IDeg 200 U/mL and IDeg 100 U/mL (both in combination with OAD(s)) after 22 weeks of treatment to a non-inferiority limit of 0.4%. The primary endpoint was change from baseline in HbA1c (%-points) after 22 weeks of treatment.

The supportive secondary endpoints included 9-point SMPG profiles, frequency of participants achieving HbA_{1c} <7%, and Health-Related Quality of Life (HRQoL; Short Form 36 [SF-36 v.2] questionnaire) scores. The safety assessments were adverse events, hypoglycaemic episodes, insulin dose, body weight, physical examination, vital signs, fundoscopy, electrocardiogram (ECG) and laboratory tests (including antibodies). Confirmed hypoglycaemic episodes were defined as episodes with a plasma glucose (PG) value of <3.1 mmol/L (56 mg/dL) and severe episodes requiring assistance. Hypoglycaemic episodes occurring between 00:01 h and 05:59 h (inclusive) were classified as nocturnal.

### Statistical Analysis

Analyses of all efficacy endpoints were based on the full analysis set (FAS) which included all randomized participants. The safety analysis set included all participants who received at least one dose of the investigational product or the comparator. Missing values were imputed using the last observation carried forward (LOCF) method.

The primary objective of this trial was to confirm efficacy of IDeg 200 U/mL in terms of glycaemic control as assessed by HbA_{1c}. This was to be assessed by comparing the difference in change from baseline in HbA_{1c} between IDeg 200 U/mL and IDeg 100 U/mL, both in combination with OAD(s), after 22 weeks of treatment to a non-inferiority limit of 0.4%.

Treatment difference in change from baseline in HbA_{1c} after 22 weeks was analyzed using an Analysis of Variance (ANOVA) model with treatment, antidiabetic treatment at screening, sex and region (Europe, North America, or South Africa) as fixed factors, and age and baseline HbA_{1c} as covariates. Responder (HbA_{1c}) analysis was based on a logistic regression model using treatment, antidiabetic therapy at screening, sex and region as fixed factors, and age and baseline HbA_{1c} as covariates. The number of treatment-emergent confirmed hypoglycaemic episodes was analyzed using a negative binomial regression model including treatment, antidiabetic therapy at screening, sex and region as fixed factors, and age as covariate. A similar model was used for analysis of treatment-emergent confirmed hypoglycaemic episodes in participants requiring ≥60 U and ≥80 U of insulin by the end of trial. However, nocturnal confirmed hypoglycaemic episodes for participants requiring ≥80 U by the end of trial was analyzed using a Poisson model with only treatment as a fixed factor.

### Results - Efficacy

Considering the whole population of patients in the trial, after 22 weeks of treatment, IDeg U-200 and IDeg U-100 effectively improved long-term glycaemic control as measured by HbA_{1c}, and IDeg U-200 was non-inferior to IDeg U-100.

The observed mean (SD) HbA_{1c} was 7.3% (1.0) with IDeg U-200 and 7.5% (0.9) with IDeg U-100. The observed mean (SD) HbA_{1c} change from baseline to end of trial was -0.79 (0.89) %-points with IDeg U-200 and -0.70 (0.90) %-points with IDeg U-100.

Both IDeg U-200 and IDeg U-100 effectively improved glycaemic control. IDeg U-200 was non-inferior to IDeg U-100 in terms of lowering HbA_{1c}, as the upper limit of the 95% confidence interval for the estimated mean treatment difference was 0.05%, which is less than the predefined non-inferiority limit of 0.4%.

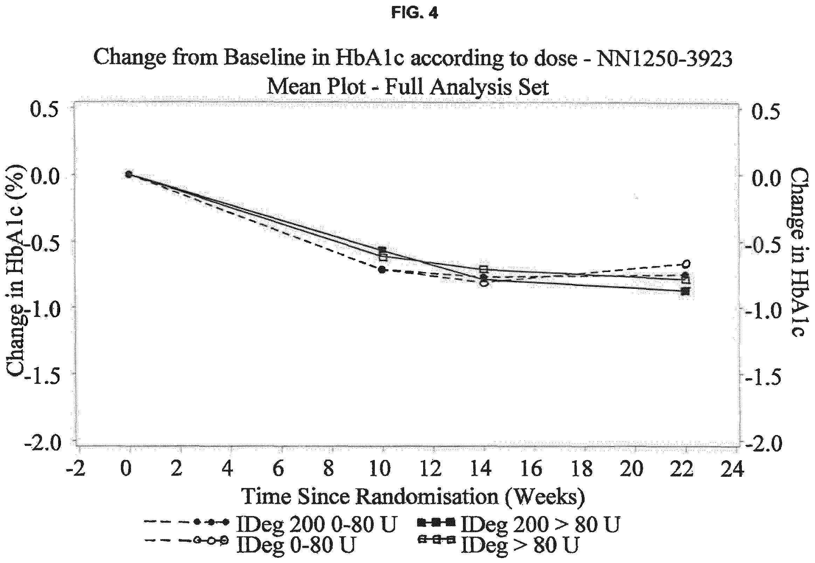

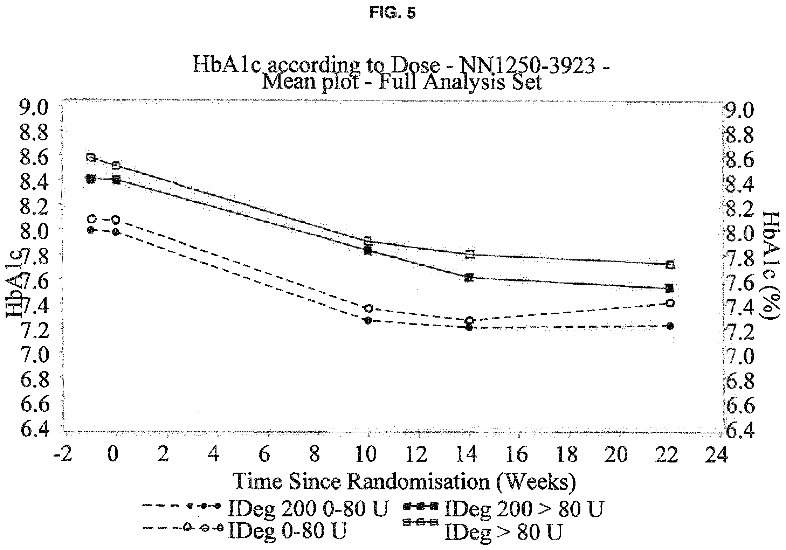

Looking into subgroups of patients receiving either up to 80 units or above 80 units of delivered insulin, HbA_{1c} reductions for subjects receiving more than 80 units were similar or tended to be greater for subjects treated with IDeg U-200 than comparator IDeg U-100 as shown in **Table 6** and **Table 7** below and corresponding graphs in **Figure 4** and **Figure 5****.**

**Table 6. Change from Baseline in HbA_{1c} according to dose - Full Analysis Set**

| | | IDeg 200 U/mL OD | | IDeg 100 U/mL OD | |
|---|---|---|---|---|---|
| | Week | N | Mean | N | Mean |
| End of trial dose 0-80 U | 0 | 113 | 0.00 | 115 | 0.00 |
| | 10 | 113 | -0.71 | 115 | -0.71 |
| | 14 | 113 | -0.77 | 115 | -0.81 |
| | 22 | 113 | -0.75 | 115 | -0.67 |
| End of trial dose > 80 U | 0 | 71 | 0.00 | 70 | 0.00 |
| | 10 | 71 | -0.57 | 70 | -0.61 |
| | 14 | 71 | -0.79 | 70 | -0.71 |
| | 22 | 71 | -0.87 | 70 | -0.78 |

| | | | | | |
|---|---|---|---|---|---|
| N: Number of subjects in FAS | | | | | |

**Table 7. HbA_{1c} according to dose - summary - Full Analysis Set**

| | | IDeg 200 U/mL OD | | IDeg 100 U/mL OD | |
|---|---|---|---|---|---|
| | Week | N | Mean | N | Mean |
| End of trial dose 0-80 U | -1 | 113 | 7.99 | 115 | 8.07 |
| | 0 | 113 | 7.97 | 115 | 8.07 |
| | 10 | 113 | 7.26 | 115 | 7.36 |
| | 14 | 113 | 7.21 | 115 | 7.26 |
| | 22 | 113 | 7.22 | 70 | 7.41 |
| End of trial dose > 80 U | -1 | 71 | 8.40 | 70 | 8.58 |
| | 0 | 71 | 8.40 | 70 | 8.51 |
| | 10 | 71 | 7.83 | 70 | 7.90 |
| | 14 | 71 | 7.61 | 115 | 7.80 |
| | 22 | 71 | 7.53 | 115 | 7.73 |

| | | | | | |
|---|---|---|---|---|---|
| N: Number of subjects in FAS | | | | | |

### Results - Safety

Similar proportions (approximately one-half) of the subjects in the treatment groups had no confirmed hypoglycaemic episodes during the trial. There were no statistically significant differences between the treatment groups in the rates of confirmed hypoglycaemic episodes and nocturnal confirmed hypoglycaemic episodes. Only 2 subjects (1 in each treatment group) had a severe hypoglycaemic episode during the trial; none of these were nocturnal. The rate of confirmed hypoglycaemic episodes and nocturnal confirmed hypoglycaemic episodes was constant throughout the trial.
Overall, the safety profile of IDeg U-200 was comparable to that of IDeg U-100.

The overall percentage of participants who experienced one or more confirmed hypoglycaemic episodes during the treatment period was 54.9% with IDeg U-200 and 52.4% with IDeg U-100 **(Table 8).** The event rates of confirmed hypoglycaemia overall with IDeg U-200 and IDeg U-100 were 5.17 and 5.66 episodes/patient-year, respectively.

Analysed by dosing group, the results show that the percentage of participants who experienced one or more confirmed hypoglycaemic episodes during the treatment period was lower in those patients receiving >80 U daily of a long acting insulin analogue by trial end than those patients receiving up to 80 U daily. Furthermore, the rate of confirmed hypoglycaemic episodes was much lower in the groups receiving >80 U daily of a long acting insulin analogue by end of trial than those patients which were administered up to 80 U daily. In particular, when IDeg U-00 was administered, in those patients receiving >80 U daily at the end of the trial, confirmed hypoglycaemic episodes were experienced in 33.8% of patients compared to 68.1% of those patients receiving 0-80 U daily. Furthermore, the rate of episodes per exposure year was 1.43 in the >80 U patient group which was lower than that of the 0-80U patient group which showed a rate of 7.54 episodes per exposure year (Table 8).
Similarly, when IDeg U-100 was administered, in those patients receiving >80 U daily at the end of the trial, confirmed hypoglycaemic episodes were experienced in 37.1% of patients compared to 62.6% of those patients receiving 0-80 U daily. Furthermore, the rate of episodes per exposure year was 3.40 in the >80 U patient group, lower than that of the 0-80 U patient group which showed a rate of 7.06 episodes per exposure year. (Table 8)
A total of 26.1% and 27.3% of participants in the IDeg U-200 and IDeg U-100 treatment groups, respectively, experienced nocturnal confirmed hypoglycaemic episodes with rates of 1.27 and 1.70 episodes/patient-year, respectively.

Analysed by dosing group, the results show that the percentage of participants who experienced one or more confirmed nocturnal hypoglycaemic episode during the treatment period was lower in the those patients receiving >80 U daily of the long acting insulin analogue by end of trial than those patients receiving up to 80 U daily. Furthermore, the rate of nocturnal confirmed hypoglycaemic episodes was much lower in the groups receiving >80 U daily of a long acting insulin analogue than those patients which were administered up to 80 U daily.
In particular, when IDeg U-200 was administered, in those patients receiving >80 U daily at the end of the trial, nocturnal confirmed hypoglycaemic episodes were experienced in 11.3% of patients compared to 35.4% of those patients receiving 0-80 U daily. Furthermore, the rate of episodes per exposure year was 0.31 in the >80 U patient group and lower than that of the 0-80 U patient group which showed a rate of 1.88 episodes per exposure year. (Table 8) Similarly, when IDeg U-100 was administered, in those patients receiving >80 U daily at the end of the trial, confirmed hypoglycaemic episodes were experienced in 15.7% of patients compared to 34.8% of those patients receiving ≤80 U daily. Furthermore, the rate of episodes per exposure year was 1.00 in the >80 U patient group and lower than that of the 0-80 U patient group which showed a rate of 2.13 episodes per exposure year. (Table 8)

**Table 8. Observed episodes of Hypoglycaemia per year of exposure**

| **Confirmed Hypoglycaemic Episodes** | **Insulin analogue** | **SAS** | **N** | **%** | **E** | **R** |
|---|---|---|---|---|---|---|
| All | IDeg 200 U/mL | 184 | 101 | 54.89 | 382 | 5.17 |
| | IDeg 100 U/mL | 187 | 98 | 52.41 | 430 | 5.66 |
| | Total | 371 | 199 | 53.64 | 812 | 5.42 |
| End of trial dose 0-80U | IDeg 200 U/mL | 113 | 77 | 68.14 | 341 | 7.54 |
| | IDeg 100 U/mL | 115 | 72 | 62.61 | 331 | 7.06 |
| | Total | 228 | 149 | 65.35 | 672 | 7.29 |
| End of trial dose >80U | IDeg 200 U/mL | 71 | 24 | 33.80 | 41 | 1.43 |
| | IDeg 100 U/mL | 70 | 26 | 37.14 | 99 | 3.40 |
| | Total | 141 | 50 | 35.46 | 140 | 2.43 |

| **Nocturnal Confirmed Hypoglycaemic Episodes** | | | | | | |
|---|---|---|---|---|---|---|
| All | IDeg 200 U/mL | 184 | 48 | 26.09 | 94 | 1.27 |
| | IDeg 100 U/mL | 187 | 51 | 27.27 | 129 | 1.70 |
| | Total | 371 | 99 | 26.68 | 223 | 1.49 |
| End of trial dose 0-80U | IDeg 200 U/mL | 113 | 40 | 35.40 | 85 | 1.88 |
| | IDeg 100 U/mL | 115 | 40 | 34.78 | 100 | 2.13 |
| | Total | 228 | 80 | 35.09 | 185 | 2.01 |
| End of trial dose >80U | IDeg 200 U/mL | 71 | 8 | 11.27 | 9 | 0.31 |
| | IDeg 100 U/mL | 70 | 11 | 15.71 | 29 | 1.00 |
| | Total | 141 | 19 | 13.48 | 38 | 0.66 |

| | | | | | | |
|---|---|---|---|---|---|---|
| SAS: Number of subjects in safety analysis set N: number of subjects experiencing at least one episode %: Percentage of subjects experiencing at least one episode E: Number of episodes R: Rate of episodes per exposure year | | | | | | |

As shown in Table 9, the estimated rate of nocturnal hypoglycaemic episodes per 100 years is lower in the patient group whom were receiving an end of trial dose of greater than 80 U. Furthermore, as can be seen from the estimate rate ratio, surprisingly IDeg U-200 was particularly effective in reducing confirmed hypoglycaemic events compared to IDeg U-100.

**Table 9. Estimated rates of hypoglycaemic episodes per 100 years of exposure**

| **End of Trial Dose** | **Subgroup of Hypoglycaemia** | **Estimated Rate of Episodes per 100 year** | | **Estimated Rate-Ratio IDeg 200U/ml / IDeg 100U/ml** | |
|---|---|---|---|---|---|
| | | **IDeg 200U/ml** | **IDeg 100U/ml** | **RR** | **95% CI** |
| 0-80U | Confirmed Hypoglycaemia | 672.5 | 575.0 | 1.170 | [0.791; 1.729] |
| | Nocturnal Confirmed Hypoglycaemia | 190.0 | 176.6 | 1.076 | [0.611; 1.893] |
| >80U | Confirmed Hypoglycaemia | 85.5 | 181.2 | 0.472 | [0.236; 0.943] |
| | Nocturnal Confirmed | 30.1 | 71.7 | 0.420 | [0.134; 1.316] |
| | Hypoglycaemia | | | | |

| | | | | | |
|---|---|---|---|---|---|
| The number of episodes is analysed using a negative binomial regression model using a log link and the logarithm of the exposure time as offset. The model includes treatment, sex, region and antidiabetic treatment at screening as fixed effects and age as covariate. For nocturnal hypos and dose >80U a Poisson model was used with treatment as fixed effect due to very sparse data. | | | | | |

The treatment with IDeg U-200 was surprisingly found to lower the risk of confirmed hypoglycaemia compared to IDeg U-100 at comparable or better glycaemic control in subjects needing high daily insulin doses of >80 U.
Thus, IDeg U-200 is particularly beneficial for patients in need of high doses because compared to IDeg U-100 treatment, treatment with IDeg U-200 provides to these group of patients both the best improvement of glycaemic control (largest change from baseline and lowest end of trial HbA_{1c}) and the lowest risk of hypoglycaemic episodes.

### Results - Dosing

At the end of the trial, approximately 38% of all subjects required a daily IDeg dose of >80 U and approximately 6% of all subjects required >160 U. With the insulin delivery systems used in this trial, the maximum doses with one single injection of IDeg 100 U/mL and IDeg 200 U/mL were 80 U and 160 U, respectively. This means that one-third of the subjects in this trial (those requiring >80 U but ≤160 U) would potentially benefit from the convenience of one single daily injection of IDeg 200 U/mL.

### Conclusion

Treatment with insulin degludec U-200 results in similar HbA_{1c} reductions as IGlar and IDegU100 from baseline, with significantly better FPG reductions and no increase in hypoglycaemia.

It has been found that long acting insulin analogues of the invention lead to a reduced risk of hypoglycaemic events in patients when administered at greater than 80 U. Furthermore, IDeg U-200 has been shown to be particularly advantageous, especially with regard to reducing the risk of hypoglycaemic events

The development of a concentrated (U-200) formulation of IDeg has the added benefit of the ability to administer larger doses of insulin in a single injection from a pre-filled pen device, thus addresses an unmet need for the growing population of overweight, obese, or severely insulin-resistant patients with T2DM who require larger doses of insulin.

The results observed with subjects using a dose > 80 units at end of trial confirm that contrary to treatment with other long acting insulin analogues the treatment with IDeg U-200 may be associated with the benefit of a lower risk of confirmed hypoglycaemia at comparable or better glycaemic control in subjects needing high daily insulin doses.

### PHARMACOLOGICAL METHODS

### Assay (I): Insulin receptor binding

The section Pharmacological studies, Assay (I) of WO2005/012347 is referenced.

### Assay (II): Potency

The section Pharmacological studies, Assay (II) of WO2005/012347 is referenced.

### References

1. Obesity Society: Your weight and diabetes. http://www.obesity.org/resources-for/your-weight-and-diabetes.htm (accessed February 9, 2012)
2. Inzucchi SE, Bergenstal RM, Buse JB, Diamant M, Ferrannini E, Nauck M, Peters AL, Tsapas A, Wender R, Matthews DR. Management of hyperglycaemia in type 2 diabetes: a patient-centered approach. Position statement of the American Diabetes Association (ADA) and the European Association for the Study of Diabetes (EASD). Diabetologia. 2012 Jun;55(6):1577-96
3. Lane WS, Cochran EK, Jackson JA, Scism-Bacon JL, Corey IB, Hirsch IB, Skyler JS. High-dose insulin therapy: is it time for U-500 insulin? Endocr Pract. 2009 Jan-Feb;15(1):71-9.
4. Crasto W, Jarvis J, Hackett E, Nayyar V, McNally PG, Davies MJ, Lawrence IG. Insulin U-500 in severe insulin resistance in type 2 diabetes mellitus. Postgrad Med J. 2009 Apr;85(1002):219-22.
5. Declaration of Helsinki. Ethical principles for medical research involving human subjects. J Indian Med Assoc 2009; 107(6):403-405.
6. ICH Harmonised Tripartite Guideline: Guideline for Good Clinical Practice. J Postgrad Med 2001; 47(3):199-203.
7. Defining and Reporting Hypoglycemia in Diabetes: A report from the American Diabetes Association Workgroup on Hypoglycemia. Diabetes Care 2005; 28(5):1245-49.
8. Cryer PE: Hypoglycemia in Diabetes: Pathophysiology, Prevalence, and Prevention. American Diabetes Association, Alexandria, VA 2009
9. Marcus A. Diabetes care - insulin delivery in a changing world. Medscape J Med 2008 10(5): 120.

## Claims

1. A long acting insulin analogue for use in reducing the risk of hypoglycaemia in a patient suffering from diabetes and requiring amounts of delivered insulin of greater than 80 U/administration, wherein the long acting insulin analogue is administered once daily as a single injection to said patient in an amount of greater than 80 U/administration and in the form of a pharmaceutical composition comprising said insulin at a concentration of 200 U/mL or greater,
and wherein the long acting insulin analogue is a naturally occurring insulin or an insulin analogue having a side chain attached either to the α-amino group of the N-terminal amino acid residue of the B chain or to the ε-amino group of a Lys residue present in the B chain of the parent insulin, the side chain being of the general formula (I):
-W-X-Y-Z
wherein W is:
• an α-amino acid residue having a carboxylic acid group in the side chain which residue forms, with one of its carboxylic acid groups, an amide group together with the α-amino group of the N-terminal amino acid residue of the B chain or together with the ε-amino group of a Lys residue present in the B chain of the parent insulin;
• a chain composed of two, three or four α-amino acid residues linked together via amide bonds, which chain - via an amide bond - is linked to the α-amino group of the N-terminal amino acid residue of the B chain or to the ε-amino group of a Lys residue present in the B chain of the parent insulin, the amino acid residues of W being selected from the group of amino acid residues having a neutral side chain and amino acid residues having a carboxylic acid group in the side chain so that W has at least one amino acid residue which has a carboxylic acid group in the side chain; or
• a covalent bond from X to the α-amino group of the N-terminal amino acid residue of the B chain or to the ε-amino group of a Lys residue present in the B chain of the parent insulin;
wherein X is:
• -CO-;
• -COCH(COOH)CO-;
• -CON(CH₂COOH)CH₂CO-;
• -CON(CH₂COOH)CH₂CON(CH₂COOH)CH₂CO-;
• -CON(CH₂CH₂COOH)CH₂CH₂CO-;
• -CON(CH₂CH₂COOH)CH₂CH₂CON(CH₂CH₂COOH)CH₂CH₂CO-;
• -CONHCH(COOH)(CH₂)₄NHCO-;
• -CON(CH₂CH₂COOH)CH₂CO-; or
• -CON(CH₂COOH)CH₂CH₂CO-.
that
a) when W is an amino acid residue or a chain of amino acid residues, *via* a bond from the underscored carbonyl carbon forms an amide bond with an amino group in W, or
b) when W is a covalent bond, *via* a bond from the underscored carbonyl carbon forms an amide bond with the N-terminal α-amino group in the B chain or with the ε-amino group of a Lys residue present in the B chain of the parent insulin;
wherein Y is:
• -(CH₂)ₘ- where m is an integer in the range of 6 to 32;
• - a divalent hydrocarbon chain comprising 1, 2 or 3 -CH=CH- groups and a number of -CH₂- groups sufficient to give a total number of carbon atoms in the chain in the range of 10 to 32;
• - a divalent hydrocarbon chain of the formula -(CH₂)ᵥC₆H₄(CH₂)_{W}- wherein v and w are integers or one of them is zero so that the sum of v and w is in the range of 6 to 30; and
wherein Z is:
• -COOH;
• -CO-Asp;
• -CO-Glu;
• -CO-Gly;
• -CO-Sar;
• -CH(COOH)₂;
• -N(CH₂COOH)₂;
• -SO₃H; or
• -PO₃H;
and any Zn²⁺ complexes thereof, provided that when W is a covalent bond and X is -CO-, then Z is different from -COOH.

2. The long acting insulin analogue for the use according to claim 1, wherein the side chain -W-X-Y-Z is attached to the ε-amino group of a Lys residue present in position 29 of the B chain,
W is selected from the group consisting of α-Asp, β-Asp, α-Glu, γ-Glu, α-hGlu and δ-hGlu; preferably γ-Glu,
X is -CO
Y is a group of the formula -(CH₂)ₘ- where m is an integer in the range of from 6 to 32, from 8 to 20, from 12 to 20, or from 12-16 and/or
Z is -COOH.

3. The long acting insulin analogue for the use according to any of the preceding claims wherein Z is -COOH.

4. The long acting insulin analogue for the use according to any of the preceding claims wherein the amino acid residue at position B30 has been deleted from the parent insulin analogue or wherein the parent insulin is des(30) human insulin.

5. The long acting insulin analogue for the use according to any of the preceding claims wherein the long acting insulin analogue is selected from the list consisting of:
N^{εB29}-(N^{α}-(HOOC(CH₂)₁₄CO)-γ-Glu) des(B30) human insulin (insulin degludec);
N^{εB29}-(N^{α}-(HOOC(CH₂)₁₅CO)-γ-Glu) des(B30) human insulin;
N^{εB29}-(N^{α}-(HOOC(CH₂)₁₆CO)-γ-Glu) des(B30) human insulin;
N^{εB29}-(N^{α}-(HOOC(CH₂)₁₇CO)-γ-Glu) des(B30) human insulin;
N^{εB29}-(N^{α}-(HOOC(CH₂)₁₈CO)-γ-Glu) des(B30) human insulin;
N^{εB29}-(N^{α}-(HOOC(CH₂)₁₆CO)-γ-Glu-N-(γ-Glu)) des(B30) human insulin;
N^{εB29}-(N^{α}-(Asp-OC(CH₂)₁₆CO)-γ-Glu) des(B30) human insulin;
N^{εB29}-(N^{α}-(Glu-OC(CH₂)₁₄CO)-γ-Glu) des(B30) human insulin;
N^{εB29}-(N^{α}-(Glu-OC(CH₂)₁₄CO-) des(B30) human insulin;
N^{εB29}-(N^{α}-(Asp-OC(CH₂)₁₆CO-) des(B30) human insulin;
N^{εB29}-(N^{α}-(HOOC(CH₂)₁₆CO)-α-Glu-N-(β-Asp)) des(B30) human insulin;
N^{εB29}-(N^{α}-(Gly-OC(CH₂)₁₃CO)-γ-Glu) des(B30) human insulin;
N^{εB29}-(N^{α}-(Sar-OC(CH₂)₁₃CO)-γ-Glu) des(B30) human insulin;
N^{εB29}-(N^{α}-(HOOC(CH₂)₁₃CO)-γ-Glu) des(B30) human insulin;
(N^{εB29}-(N^{α}-(HOOC(CH₂)₁₃CO)-β-Asp) des(B30) human insulin;
N^{εB29}-(N^{α}-(HOOC(CH₂)₁₃CO)-α-Glu) des(B30) human insulin;
N^{εB29}-(N^{α}-(HOOC(CH₂)₁₆CO)-γ-D-Glu) des(B30) human insulin;
N^{εB29}-(N^{α}-(HOOC(CH₂)₁₄CO)-β-D-Asp) des(B30) human insulin;
N^{εB29}-(N-HOOC(CH₂)₁₆CO-β-D-Asp) des(B30) human insulin;
N^{εB29}-(N-HOOC(CH₂)₁₄CO-IDA) des(B30) human insulin;
N^{εB29}-[N-(HOOC(CH₂)₁₆CO)-N-(carboxyethyl)-Gly] des(B30) human insulin;
N^{εB29}-[N-(HOOC(CH₂)₁₄CO)-N-(carboxyethyl)-Gly] des(B30) human insulin; and
N^{εB29}-[N-(HOOC(CH₂)₁₄CO)-N-(carboxymethyl)-β-Ala] des(B30) human insulin.

6. The long acting insulin analogue for the use according to any of the preceding claims wherein the long acting insulin analogue is insulin degludec.

7. The long acting insulin analogue for the use according to any of the preceding claims wherein the long acting insulin analogue is administered in an amount from 80 U/administration to 160 U/administration.

8. The long acting insulin analogue for the use according to any of the preceding claims wherein the patient is suffering from type-2 diabetes.

9. The long acting insulin analogue for use according to any preceding claims wherein the patient has a body/mass index (BMI) of 30kg/m² or more.

10. The long acting insulin analogue for the use according to any of the preceding claims wherein the hypoglycaemia is nocturnal hypoglycaemia.

11. The long acting insulin analogue for the use according to any of the preceding claims wherein the risk of hypoglycaemia is reduced to a rate of hypoglycaemic episodes of less than 2 episodes/exposure in a year, of less than 1.5 episodes/exposure in a year, of less than 1 episodes/exposure in a year or of less than 0.5 episodes/exposure in a year.

12. The long acting insulin analogue for the use according to any of the preceding claims wherein the long actin insulin analogue has an insulin action for at least 24 hours.

13. The long acting insulin analogue for the use according to claim 12 wherein the long actin insulin analogue has an insulin action for at least 30 hours, at least 36 hours, at least 42 hours, at least 48 hours, between 24 and 48 hours after administration, between 30 and 48 hours after administration, between 36 and 48 hours after administration, between 24 and 42 hours after administration, between 24 and 36 hours after administration, between 30 and 42 hours after administration, or between 30 and 36 hours after administration.

## Patentansprüche

1. Lang wirkendes Insulinanalog für die Verwendung bei der Reduzierung des Risikos von Hypoglykämie in einem Patienten, der an Diabetes leidet und Mengen zugeführten Insulins von mehr als 80 U/Verabreichung benötigt, wobei das lang wirkende Insulinanalog einmal täglich als eine einzelne Injektion an den Patienten in einer Menge von mehr als 80 U/Verabreichung und in der Form einer pharmazeutischen Zusammensetzung verabreicht wird, umfassend das Insulin in einer Konzentration von 200 U/mL oder höher,
und wobei das lang wirkende Insulinanalog ein natürlich vorkommendes Insulin oder ein Insulinanalog mit einer Seitenkette, befestigt entweder an der α-Aminogruppe des N-terminalen Aminosäurerückstands der B-Kette oder an der ε-Aminogruppe eines Lys-Rückstands, der in der B-Kette des Ursprungsinsulins vorkommt, die Seitenkette bestehend aus der allgemeinen Formel (I):
-W-X-Y-Z
wobei W:
• ein α-Aminosäurerückstand ist, mit einer Carbonsäuregruppe in der Seitenkette, und der Rückstand mit einer seiner Carbonsäuregruppen eine Amidgruppe bildet, gemeinsam mit dem α-Amino des N-terminalen Aminosäurerückstands der B-Kette oder gemeinsam mit der ε-Amino-Gruppe eines Lys-Rückstands, der in der B-Kette des Ursprungsinsulins vorkommt;
• eine Kette ist, bestehend aus zwei, drei oder vier α-Aminosäurerückständen, verbunden miteinander über Amidbindungen, und die Kette - über eine Amidbindung - mit der α-Aminogruppe des N-terminalen Aminosäurerückstands der B-Kette oder mit der ε-Aminogruppe eines Lys-Rückstands verbunden ist, der in der B-Kette des Ursprungsinsulins vorkommt, die Aminosäurerückstände von W, ausgewählt aus der Gruppe von Aminosäurerückständen mit einer neutralen Seitenkette und Aminosäurerückständen mit einer Karbonsäuregruppe in der Seitenkette, sodass W zumindest einen Aminosäurerückstand bezeichnet, der eine Karbonsäuregruppe in der Seitenkette aufweist; oder
• eine kovalente Bindung von X zur α-Aminogruppe des N-terminalen Aminosäurerückstands der B-Kette oder zur ε-Aminogruppe eines Lys-Rückstands, der in der B-Kette des Ursprungsinsulins vorkommt;
wobei X so ist:
• -CO-;
• -COCH(COOH)CO-;
• -CON(CH₂COOH)CH₂CO-;
• -CON(CH₂COOH)CH₂CON(CH₂COOH)CH₂CO-;
• -CON(CH₂CH₂COOH)CH₂CH₂CO-;
• -CON(CH₂CH₂COOH)CH₂CH₂CON(CH₂CH₂COOH)CH₂CH₂CO-;
• -CONHCH(COOH)(CH₂)₄NHCO-;
• -CON(CH₂CH₂COOH)CH₂CO-; oder
• -CON(CH₂COOH)CH₂CH₂CO-.
dass
a) wenn W ein Aminosäurerückstand oder eine Kette eines Aminosäurerückstandes ist, sich *über* eine Bindung vom unterstrichenen Carbonylkarbon eine Amidbindung mit einer Aminogruppe in W bildet, oder
b) wenn W eine kovalente Bindung ist, sich *über* eine Bindung von der unterstrichenen Carbonylkarbon eine Amidbindung mit der N-terminalen α-Aminogruppe in der B-Kette oder mit der ε-Aminogruppe eines Lys-Rückstands, der in der B-Kette des Ursprungsinsulins vorkommt;
wobei Y so ist:
• -(CH₂)ₘ- wobei m ein Integer zwischen 6 und 32 ist;
• - eine zweiwertige Kohlenwasserstoffkette umfassend 1, 2 oder 3 -CH=CH- Gruppen und eine Anzahl an -CH₂- Gruppen, ausreichend, um eine Gesamtanzahl an Kohlenstoffatomen in der Kette in der Bandbreite von 10 bis 32 zu erreichen;
• - eine zweiwertige Kohlenwasserstoffkette der Formel-(CH₂)ᵥC₆H₄(CH₂)w- wobei v und w Integer sind oder eine von ihnen Null beträgt, sodass die Summe von v und w sich zwischen 6 und 30 bewegt; und wobei Z ist:
• -COOH;
• -CO-Asp;
• -CO-Glu;
• -CO-Gly;
• -CO-Sar;
• -CH(COOH)₂;
• -N(CH₂COOH)₂;
• -SO₃H; oder
• -PO₃H;
und alle Zn²⁺ Komplexe davon, vorausgesetzt, dass wenn W eine kovalente Bindung ist und X -CO- ist, Z von -COOH abweicht.

2. Lang wirkendes Insulinanalog für die Verwendung gemäß Anspruch 1, wobei die Seitenkette -W-X-Y-Z an der ε-Aminogruppe eines Lys-Rückstands angeordnet ist, der in Position 29 der B-Kette vorkommt,
W ausgewählt ist aus der Gruppe, bestehend aus α-Asp, β-Asp, α-Glu, γ-Glu, α-hGlu und δ-hGlu; bevorzugt γ-Glu,
X -CO ist
Y eine Gruppe der Formel -(CH₂)ₘ- ist. wobei m ein Integer zwischen 6 und 32, zwischen 8 und 20, zwischen 12 und 20 oder zwischen 12 - 16 ist und/oder
Z -COOH ist.

3. Lang wirkendes Insulinanalog für die Verwendung gemäß einem der vorstehenden Ansprüche, wobei Z -COOH ist.

4. Lang wirkendes Insulinanalog für die Verwendung gemäß einem der vorstehenden Ansprüche, wobei der Aminosäurerückstand an Position B30 vom Ursprungsinsulinanalog gelöscht wurde oder wobei das Ursprungsinsulin des(30) menschliches Insulin ist.

5. Lang wirkendes Insulinanalog für die Verwendung gemäß einem der vorstehenden Ansprüche, wobei das lang wirkende Insulinanalog aus der Liste ausgewählt wird, bestehend aus:
N^{εB29}-(N^{α}-(HOOC(CH₂)₁₄CO)-γ-Glu) des (B30) menschlichem Insulin (Degludec-Insulin) ;
N^{εB29}-(N^{α}-(HOOC(CH₂)₁₅CO)-γ-Glu) des(B30) menschlichem Insulin;
N^{εB29}-(N^{α}-(HOOC(CH₂)₁₆CO)-γ-Glu) des(B30) menschlichem Insulin;
N^{εB29}-(N^{α}-(HOOC(CH₂)₁₇CO)-γ-Glu) des(B30) menschlichem Insulin;
N^{εB29}-(N^{α}-(HOOC(CH₂)₁₈CO)-γ-Glu) des(B30) menschlichem Insulin;
N^{εB29}-(N^{α}-(HOOC(CH₂)₁₆CO)-γ-Glu-N-(γ-Glu)) des (B30) menschlichem Insulin;
N^{εB29}-(N^{α}-(Asp-OC(CH₂)₁₆CO)-γ-Glu) des(B30) menschlichem Insulin;
N^{εB29}-(N^{α}-(Glu-OC(CH₂)₁₄CO)-γ-Glu) des(B30) menschlichem Insulin;
N^{εB29}-(N^{α}-(Glu-OC(CH₂)₁₄CO-) des(B30) menschlichem Insulin;
N^{εB29}-(N^{α}-(Asp-OC(CH₂)₁₆CO-) des(B30) menschlichem Insulin;
N^{εB29}-(N^{α}-(HOOC(CH₂)₁₆CO)-α-Glu-N-(β-Asp)) des (B30) menschlichem Insulin;
N^{εB29}-(N^{α}-(Gly-OC(CH₂)₁₃CO)-γ-Glu) des(B30) menschlichem Insulin;
N^{εB29}-(N^{α}-(Sar-OC(CH₂)₁₃CO)-γ-Glu) des(B30) menschlichem Insulin;
N^{εB29}-(N^{α}-(HOOC(CH₂)₁₃CO)-γ-Glu) des(B30) menschlichem Insulin;
(N^{εB29}-(N^{α}-(HOOC(CH₂)₁₃CO)-β-Asp) des(B30) menschlichem Insulin;
N^{εB29}-(N^{α}-(HOOC(CH₂)₁₃CO)-α-Glu) des(B30) menschlichem Insulin;
N^{εB29}-(N^{α}-(HOOC(CH₂)₁₆CO)-γ-D-Glu) des(B30) menschlichem Insulin;
N^{εB29}-(N^{α}-(HOOC(CH₂)₁₄CO)-β-D-Asp) des(B30) menschlichem Insulin;
N^{εB29}-(N-HOOC(CH₂)₁₆CO-β-D-Asp) des(B30) menschlichem Insulin;
N^{εB29}-(N-HOOC(CH₂)₁₄CO-IDA) des(B30) menschlichem Insulin;
N^{εB29}-[N-(HOOC(CH₂)₁₆CO)-N-(Carboxyethyl)-Gly] des (B30) menschlichem Insulin;
N^{εB29}-[N-(HOOC(CH₂)₁₄CO)-N-(Carboxyethyl) -Gly] des (B30) menschlichem Insulin; und
N^{εB29}-[N-(HOOC(CH₂)₁₄CO)-N-(Carboxymethyl)-β-Ala] des (B30) menschlichem Insulin.

6. Lang wirkendes Insulinanalog für die Verwendung gemäß einem der vorstehenden Ansprüche, wobei das lang wirkende Insulinanalog Degludec-Insulin ist.

7. Lang wirkendes Insulinanalog für die Verwendung gemäß einem der vorstehenden Ansprüche, wobei das lang wirkende Insulinanalog in einer Menge zwischen 80 U/Verabreichung und 160 U/Verabreichung verabreicht wird.

8. Lang wirkendes Insulinanalog für die Verwendung gemäß einem der vorstehenden Ansprüche, wobei der Patient an Typ-II-Diabetes leidet.

9. Das lang wirkende Insulinanalog für die Verwendung gemäß einem der vorstehenden Ansprüche, wobei der Patient einen BMI (Körpermaßindex) von 30kg/m² oder mehr hat.

10. Lang wirkendes Insulinanalog für die Verwendung gemäß einem der vorstehenden Ansprüche, wobei die Hypoglykämie nächtliche Hypoglykämie ist.

11. Lang wirkendes Insulinanalog für die Verwendung gemäß einem der vorstehenden Ansprüche, wobei das Risiko von Hypoglykämie bis zu einer Rate von hypoglykämischen Episoden von weniger als 2 Episoden/Einwirkung in einem Jahr, von weniger als 1,5 Episoden/Einwirkung in einem Jahr, von weniger als 1 Episode/Einwirkung in einem Jahr oder von weniger als 0,5 Episoden/Einwirkung in einem Jahr.

12. Lang wirkendes Insulinanalog für die Verwendung gemäß einem der vorstehenden Ansprüche, wobei das lang wirkende Insulinanalog über eine Insulinwirkung verfügt, die zumindest 24 Stunden andauert.

13. Lang wirkendes Insulinanalog für die Verwendung gemäß Anspruch 12, wobei das lang wirkende Insulinanalog eine Insulinwirkung von zumindest 30 Stunden hat, zumindest 36 Stunden, zumindest 42 Stunden, zumindest 48 Stunden, zwischen 24 und 48 Stunden nach Verabreichung, zwischen 30 und 48 Stunden nach Verabreichung, zwischen 36 und 48 Stunden nach Verabreichung, zwischen 24 und 42 Stunden nach Verabreichung, zwischen 24 und 36 Stunden nach Verabreichung, zwischen 30 und 42 Stunden nach Verabreichung, oder zwischen 30 und 36 Stunden nach Verabreichung.

## Revendications

1. Analogue de l'insuline à action prolongée pour l'utilisation destinée à réduire le risque d'hypoglycémie chez un patient souffrant de diabètes et nécessitant des quantités d'insuline administrée supérieure à 80 U/administration, dans lequel l'analogue de l'insuline à action prolongée est administré une fois par jour en une seule injection audit patient en une quantité supérieure à 80 U/administration et sous la forme d'une composition pharmaceutique comprenant ladite insuline à une concentration de 200 U/mL ou supérieure,
et dans lequel l'analogue de l'insuline à action prolongée est de l'insuline d'origine naturelle ou un analogue de l'insuline ayant une chaîne latérale attachée soit au groupe α-aminé du résidu d'acide aminé N terminal de la chaîne B soit au groupe ε-aminé d'un résidu Lys présent dans la chaîne B de l'insuline parente, la chaîne latérale étant de la formule générale (I) :
-W-X-Y-Z
dans lequel W est :
• un résidu d'acide α-aminé ayant un groupe acide carboxylique dans la chaîne latérale qui forme des résidus, avec un de ses groupes acide carboxylique, un groupe amide ensemble avec le groupe α-aminé du résidu d'acide aminé N terminal de la chaîne B ou ensemble avec le groupe ε-aminé d'un résidu Lys présent dans la chaîne B de l'insuline parente ;
• une chaîne composée de deux, trois ou quatre résidus d'acide α-aminé liés ensemble via des liaisons amides, cette chaîne - via une liaison amide - est liée au groupe α-aminé du résidu d'acide aminé N terminal de la chaîne B ou au groupe ε-aminé d'un résidu Lys présent dans la chaîne B de l'insuline parente, les résidus d'acides aminés de W étant choisis parmi le groupe constitué de résidus d'acides aminés ayant une chaîne latérale neutre et des résidus d'acides aminés ayant un groupe acide carboxylique dans la chaîne latérale de sorte que W présente au moins un résidu d'acide aminé qui a un groupe acide carboxylique dans la chaîne latérale ; ou
• une liaison covalente de X au groupe α-aminé du résidu d'acide aminé N terminal de la chaîne B ou au groupe ε-aminé d'un résidu Lys présent dans la chaîne B de l'insuline parente ;
dans lequel X est :
• -CO- ;
• -COCH(COOH)CO- ;
• -CON(CH₂COOH)CH₂CO- ;
• -CON(CH₂COOH)CH₂CON(CH₂COOH)CH₂CO- ;
• -CON(CH₂CH₂COOH)CH₂CH₂CO- ;
• -CON(CH₂CH₂COOH)CH₂CH₂CON(CH₂CH₂COOH)CH₂CH₂CO- ;
• -CONHCH(COOH)(CH₂)₄NHCO- ;
• -CON(CH₂CH₂COOH)CH₂CO- ; ou
• -CON(CH₂COOH)CH₂CH₂CO-.
que
a) lorsque W est un résidu d'acide aminé ou une chaîne de résidus d'acides aminés, *via* une liaison du carbone carbonyle souligné forme une liaison amide avec un groupe aminé dans W, ou
b) lorsque W est une liaison covalente, *via* une liaison du carbone carbonyle souligné forme une liaison amide avec le groupe α-aminé N terminal dans la chaîne B ou avec le groupe ε-aminé d'un résidu Lys présent dans la chaîne B de l'insuline parente ;
dans lequel Y est :
• -(CH₂)ₘ- où m est un nombre entier compris dans la gamme allant de 6 à 32 ;
• - une chaîne d'hydrocarbures divalents comprenant 1, 2 ou 3 groupes -CH=CH- et une quantité de groupes-CH₂- suffisants pour donner une quantité totale d'atomes de carbone dans la chaîne dans la gamme de 10 à 32 ;
• - une chaîne d'hydrocarbures divalents de la formule -(CH₂)ᵥC₆H₄(CH₂)w- dans lequel v et w sont des nombres entiers ou l'un d'eux est zéro de sorte que la somme de v et w se trouve dans la gamme de 6 à 30 ; et dans lequel Z est :
• -COOH ;
• -CO-Asp ;
• -CO-Glu ;
• -CO-Gly ;
• -CO-Sar ;
• -CH(COOH)₂ ;
• -N(CH₂COOH)₂ ;
• -SO₃H ; ou
• -PO₃H ;
et tout complexe Zn²⁺ de ceux-ci, à condition que lorsque W est une liaison covalente et X est -CO-, Z est alors différent de -COOH.

2. Analogue de l'insuline à action prolongée pour l'utilisation selon la revendication 1, dans lequel la chaîne latérale -W-X-Y-Z est attachée au groupe ε-aminé d'un résidu Lys présent dans la position 29 de la chaîne B,
W est choisi parmi le groupe constitué de α-Asp, β-Asp, α-Glu, γ-Glu, α-hGlu et δ-hGlu ; de préférence γ-Glu,
X est -CO
Y est un groupe de la formule -(CH₂)ₘ- où m est un nombre entier compris dans la gamme allant de 6 à 32, allant de 8 à 20, allant de 12 à 20 ou allant de 12-16 et/ou Z est -COOH.

3. Analogue de l'insuline à action prolongée pour l'utilisation selon l'une quelconque des revendications précédentes, dans lequel Z est -COOH.

4. Analogue de l'insuline à action prolongée pour l'utilisation selon l'une quelconque des revendications précédentes, dans lequel le résidu d'acide aminé au niveau de la position B30 a été éliminé de l'analogue de l'insuline parente ou dans lequel l'insuline parente est des(30) de l'insuline humaine.

5. Analogue de l'insuline à action prolongée pour l'utilisation selon l'une quelconque des revendications précédentes, dans lequel l'analogue de l'insuline à action prolongée est choisi parmi la liste constituée de :
N^{εB29}-(N^{α}-(HOOC(CH₂)₁4CO)-γ-Glu) des (B30) insuline humaine (insuline dégludec) ;
N^{εB29}-(N^{α}-(HOOC(CH₂)₁₅CO)-γ-Glu) des (B30) insuline humaine ;
N^{εB29}-(N^{α}-(HOOC(CH₂)₁₆CO)-γ-Glu) des (B30) insuline humaine ;
N^{εB29}-(N^{α}-(HOOC(CH₂)₁₇CO)-γ-Glu) des (B30) insuline humaine ;
N^{εB29}-(N^{α}-(HOOC(CH₂)₁₈CO)-γ-Glu) des (B30) insuline humaine ;
N^{εB29}-(N^{α}-(HOOC(CH₂)₁₆CO)-γ-Glu-N-(γ-Glu)) des (B30) insuline humaine ;
N^{εB29}-(N^{α}-(Asp-OC(CH₂)₁₆CO)-γ-Glu) des (B30) insuline humaine ;
N^{εB29}-(N^{α}-(Glu-OC(CH₂)₁₄CO)-γ-Glu) des (B30) insuline humaine ;
N^{εB29}-(N^{α}-(Glu-OC(CH₂)₁₄CO-) des (B30) insuline humaine ;
N^{εB29}-(N^{α}-(Asp-OC(CH₂)₁₆CO-) des (B30) insuline humaine ;
N^{εB29}-(N^{α}-(HOOC(CH₂)₁₆CO)-α-Glu-N-(β-Asp)) des (B30) insuline humaine ;
N^{εB29}-(N^{α}-(Gly-OC(CH₂)₁₃CO)-γ-Glu) des (B30) insuline humaine ;
N^{εB29}-(N^{α}-(Sar-OC(CH₂)₁₃CO)-γ-Glu) des (B30) insuline humaine ;
N^{εB29}-(N^{α}-(HOOC(CH₂)₁₃CO)-γ-Glu) des (B30) insuline humaine ;
(N^{εB29}-(N^{α}-(HOOC(CH₂)₁₃CO)-β-Asp) des (B30) insuline humaine ;
N^{εB29}-(N^{α}-(HOOC(CH₂)₁₃CO)-α-Glu) des (B30) insuline humaine ;
N^{εB29}-(N^{α}-(HOOC(CH₂)₁₆CO)-γ-D-Glu) des (B30) insuline humaine ;
N^{εB29}-(N^{α}-(HOOC(CH₂)₁₄CO)-β-D-Asp) des (B30) insuline humaine ;
N^{εB29}-(N-HOOC(CH₂)₁₆CO-β-D-Asp) des(B30) insuline humaine ;
N^{εB29}-(N-HOOC(CH₂)₁₄CO-IDA) des(B30) insuline humaine ;
N^{εB29}-[N-(HOOC(CH₂)₁₆CO)-N-(carboxyethyl)-Gly] des (B30) insuline humaine ;
N^{εB29}-[N-(HOOC(CH₂)₁₄CO)-N-(carboxyethyl)-Gly] des (B30) insuline humaine ; et
N^{εB29}-[N-(HOOC(CH₂)₁₄CO)-N-(carboxymethyl)-β-Ala] des (B30) insuline humaine.

6. Analogue de l'insuline à action prolongée pour l'utilisation selon l'une quelconque des revendications précédentes, dans lequel l'analogue de l'insuline à action prolongée est l'insuline dégludec.

7. Analogue de l'insuline à action prolongée pour l'utilisation selon l'une quelconque des revendications précédentes, dans lequel l'analogue de l'insuline à action prolongée est administré en une quantité allant de 80 U/administration à 160 U/administration.

8. Analogue de l'insuline à action prolongée pour l'utilisation selon l'une quelconque des revendications précédentes, dans lequel le patient souffre de diabètes de type 2.

9. Analogue de l'insuline à action prolongée pour l'utilisation selon l'une quelconque des revendications précédentes, dans lequel le patient a un indice de masse corporelle (IMC) de 30kg/m² ou davantage.

10. Analogue de l'insuline à action prolongée pour l'utilisation selon l'une quelconque des revendications précédentes, dans lequel l'hypoglycémie est de l'hypoglycémie nocturne.

11. Analogue de l'insuline à action prolongée pour l'utilisation selon l'une quelconque des revendications précédentes, dans lequel le risque d'hypoglycémie est réduit à un taux d'épisodes hypoglycémiques inférieurs à 2 épisodes/exposition en un an, inférieurs à 1,5 épisodes/exposition en un an, inférieurs à 1 épisode/exposition en un an ou inférieurs à 0,5 épisode/exposition en un an.

12. Analogue de l'insuline à action prolongée pour l'utilisation selon l'une quelconque des revendications précédentes, dans lequel l'analogue de l'insuline à action prolongée a une action de l'insuline pour au moins 24 heures.

13. Analogue de l'insuline à action prolongée pour l'utilisation selon la revendication 12, dans lequel l'analogue de l'insuline à action prolongée a une action de l'insuline pour au moins 30 heures, au moins 36 heures, au moins 42 heures, au moins 48 heures, entre 24 et 48 heures après l'administration, entre 30 et 48 heures après l'administration, entre 36 et 48 heures après l'administration, entre 24 et 42 heures après l'administration, entre 24 et 36 heures après l'administration, entre 30 et 42 heures après l'administration ou entre 30 et 36 heures après l'administration.
